(19) Europäisches Patentamt
European Patent Office
Office européen des brevets

(11) EP 3 124 607 A1

(12) # EUROPEAN PATENT APPLICATION

published in accordance with Art. 153(4) EPC

(43) Date of publication:
**01.02.2017 Bulletin 2017/05**

(21) Application number: **15769869.7**

(22) Date of filing: **27.03.2015**

(51) Int Cl.:
***C12N 15/00*** *(2006.01)*
***A61K 45/00*** *(2006.01)*
***A61P 43/00*** *(2006.01)*
***C12Q 1/68*** *(2006.01)*
***G01N 33/566*** *(2006.01)*
***A61K 31/7088*** *(2006.01)*
***A61K 48/00*** *(2006.01)*
***C12N 15/115*** *(2010.01)*
***G01N 33/53*** *(2006.01)*

(86) International application number:
**PCT/JP2015/059732**

(87) International publication number:
**WO 2015/147290 (01.10.2015 Gazette 2015/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **27.03.2014 JP 2014067289**

(71) Applicant: **RIBOMIC INC.**
**Minato-ku**
**Tokyo 108-0071 (JP)**

(72) Inventors:
- **IKEDA, Hisako**
  **Tokyo 108-0071 (JP)**
- **MIYAKAWA, Shin**
  **Tokyo 108-0071 (JP)**

(74) Representative: **Campbell, Patrick John Henry**
**J A Kemp**
**14 South Square**
**Gray's Inn**
**London WC1R 5JJ (GB)**

(54) **APTAMER INHIBITING BIOLOGICAL ACTIVITY OF AUTOTAXIN BY BINDING WITH AUTOTAXIN, AND USE THEREOF**

(57) The present invention provides an aptamer that binds to an autotaxin, which contains a nucleotide sequence represented by the following formula (I):

CGGAACC-$N_1$-GGTC (I)

wherein $N_1$ shows any of 3 to 11 nucleotides, and a utilization method thereof.

EP 3 124 607 A1

SEQUENCE LISTING

<110>  RIBOMIC INC.

<120>  Aptamer binding to autotaxin and inhibiting its physiological activity and use thereof

<130>  092320

<150>  JP 2014-067289
<151>  2014-03-27

<160>  30

<170>  PatentIn version 3.5

<210>  1
<211>  74
<212>  DNA
<213>  Artificial Sequence

<220>
<223>  DNA random pool sequence

<220>
<221>  misc_feature
<222>  (18)..(57)
<223>  n is a, c, g, or t

<400>  1
gtggtctagc tgtactcnnn nnnnnnnnnn nnnnnnnnnn nnnnnnnnnn nnnnnnncca      60

cagtcaacga gcta                                                        74

<210>  2
<211>  17
<212>  DNA
<213>  Artificial Sequence

<220>
<223>  Primer

<400>  2
gtggtctagc tgtactc                                                     17

<210>  3
<211>  17
<212>  DNA
<213>  Artificial Sequence

<220>
<223>  Primer

<400>  3
tagctcgttg actgtgg                                                     17

<210>  4
<211>  74
<212>  DNA

<213>  Artificial Sequence

<220>
<223>  DNA aptamer

<400>  4
gtggtctagc tgtactctcc ggaaccagag caatttggtc gagcgctatc ggatggtcca      60

cagtcaacga gcta                                                        74

<210>  5
<211>  74
<212>  DNA
<213>  Artificial Sequence

<220>
<223>  DNA aptamer

<400>  5
gtggtctagc tgtactcatg gacggaacca gaatactttt ggtctccatt gagtacgcca      60

cagtcaacga gcta                                                        74

<210>  6
<211>  75
<212>  DNA
<213>  Artificial Sequence

<220>
<223>  DNA aptamer

<400>  6
gtggtctagc tgtactcgga accgtactca acggtcagta cctttgcgcc gcagcaagcc      60

acagtcaacg agcta                                                       75

<210>  7
<211>  74
<212>  DNA
<213>  Artificial Sequence

<220>
<223>  DNA aptamer

<400>  7
gtggtctagc tgtactcgcc tgccggaacc gcccctgtgg tcgcatcgag caacggccca      60

cagtcaacga gcta                                                        74

<210>  8
<211>  74
<212>  DNA
<213>  Artificial Sequence

<220>
<223>  DNA aptamer

<400>  8
gtggtctagc tgtactccga aagccggaac cgtgccaatg gtcgctactt cagctcccca      60

cagtcaacga gcta 74

<210> 9
<211> 74
<212> DNA
<213> Artificial Sequence

<220>
<223> DNA aptamer

<400> 9
gtggtctagc tgtactcagg ccggaaccgg tgaaattggt cgcctaataa gcgaaatcca 60

cagtcaacga gcta 74

<210> 10
<211> 74
<212> DNA
<213> Artificial Sequence

<220>
<223> DNA aptamer

<400> 10
gtggtctagc tgtactcgcc ggaaccgtac tatggtcgcg ttgtatgacg cttgtatcca 60

cagtcaacga gcta 74

<210> 11
<211> 45
<212> DNA
<213> Artificial Sequence

<220>
<223> DNA aptamer

<400> 11
gtactcatgg acggaaccag aatactttg gtctccattg agtac 45

<210> 12
<211> 34
<212> DNA
<213> Artificial Sequence

<220>
<223> DNA aptamer

<400> 12
cctggacgga accagaatac ttttggtctc cagg 34

<210> 13
<211> 30
<212> DNA
<213> Artificial Sequence

<220>
<223> DNA aptamer

<400> 13
tggacggaac cagaatactt ttggtctcca 30

<210> 14
<211> 30
<212> DNA
<213> Artificial Sequence

<220>
<223> DNA aptamer

<400> 14
gggacggaac cagaatactt ttggtctccc 30

<210> 15
<211> 30
<212> DNA
<213> Artificial Sequence

<220>
<223> DNA aptamer

<400> 15
cctggacgga accaatactt ggtctccagg 30

<210> 16
<211> 32
<212> DNA
<213> Artificial Sequence

<220>
<223> DNA aptamer

<400> 16
ctggacggaa ccagaatact tttggtctcc ag 32

<210> 17
<211> 64
<212> DNA
<213> Artificial Sequence

<220>
<223> DNA random pool sequence

<220>
<221> misc_feature
<222> (18)..(47)
<223> n is a, c, g, or t

<400> 17
acactcacag gcgctggnnn nnnnnnnnnn nnnnnnnnnn nnnnnnncgt gcatggccgc 60

tagt 64

<210> 18
<211> 17

<212> DNA
<213> Artificial Sequence

<220>
<223> Primer

<400> 18
acactcacag gcgctgg 17

<210> 19
<211> 17
<212> DNA
<213> Artificial Sequence

<220>
<223> Primer

<400> 19
actagcggcc atgcacg 17

<210> 20
<211> 64
<212> DNA
<213> Artificial Sequence

<220>
<223> DNA aptamer

<400> 20
acactcacag gcgctggggt acgctcggaa ccgaggcaat tggtcagcgt gcatggccgc 60

tagt 64

<210> 21
<211> 64
<212> DNA
<213> Artificial Sequence

<220>
<223> DNA aptamer

<400> 21
acactcacag gcgctggcca ccactgcacc ggaaccgcga atgtggtcgt gcatggccgc 60

tagt 64

<210> 22
<211> 64
<212> DNA
<213> Artificial Sequence

<220>
<223> DNA aptamer

<400> 22
acactcacag gcgctggccg gaaccgtgca tatggtcgcc agcacatcgt gcatggccgc 60

tagt 64

<210> 23
<211> 64
<212> DNA
<213> Artificial Sequence

<220>
<223> DNA aptamer

<400> 23
acactcacag gcgctggcac ggaccggaac cgggacgctc ggtcgaccgt gcatggccgc 60

tagt 64

<210> 24
<211> 64
<212> DNA
<213> Artificial Sequence

<220>
<223> DNA aptamer

<400> 24
acactcacag gcgctggcga gtcggaaccg agccgattgg tcactcgcgt gcatggccgc 60

tagt 64

<210> 25
<211> 64
<212> DNA
<213> Artificial Sequence

<220>
<223> DNA aptamer

<400> 25
acactcacag gcgctggcga cgtcggaacc gtgtaccatg gtcacgtcgt gcatggccgc 60

tagt 64

<210> 26
<211> 38
<212> DNA
<213> Artificial Sequence

<220>
<223> DNA aptamer

<400> 26
cgctggggta cgctcggaac cgaggcaatt ggtcagcg 38

<210> 27
<211> 32
<212> DNA
<213> Artificial Sequence

<220>
<223> DNA aptamer

<400> 27
tacgctcgga accgaggcaa ttggtcagcg tg 32

<210> 28
<211> 31
<212> DNA
<213> Artificial Sequence

<220>
<223> DNA aptamer

<400> 28
acaggcgctg gccggaaccg tgcatatggt c 31

<210> 29
<211> 31
<212> DNA
<213> Artificial Sequence

<220>
<223> DNA aptamer

<400> 29
gctggccgga accgtgcata tggtcgccag c 31

<210> 30
<211> 11
<212> DNA
<213> Artificial Sequence

<220>
<223> SL1 sequence

<400> 30
agaatacttt t 11

## Description

[Technical Field]

The present invention relates to an aptamer for autotaxin and a utilization method thereof and the like.

[Background Art]

Autotaxin is a secretory protein identified as a molecule that promotes motility of melanoma cells. It belongs to the Enpp (ectonucleotide pyrophosphatase/phosphodiesterase) family proteins and also known as Enpp2. It has a phosphodiesterase activity and is involved in extracellular nucleotide metabolism. It also has a Lysophospholipase D activity (LysoPLD activity), and is also an enzyme that degrades lysophosphatidylcholine (LPC) into lysophosphatidic acid (LPA) and choline. Produced LPA shows various physiological activities of a lipid mediator, such as cellular motility activation, cell proliferation, angiogenesis and the like. LPA is said to be involved in the growth, metastasis and the like of cancer cells, and many studies of LPA have been made. Also, there are many reports on increased expression and activity of autotaxin, which is a LPA producing enzyme, in the blood and ascites of cancer patients.

Recently, a fibrosis suppressive effect by LPA receptor LPA1 knocked-out mouse and LPA1 inhibitors in a pulmonary fibrosis model by bleomycin induction has been reported, thus suggesting relation between LPA and pulmonary fibrosis, and autotaxin as an LPA producing enzyme is drawing attention as to the relation with pulmonary fibrosis.

Among them is a report that an anti-autotaxin monoclonal antibody has a prophylactic and/or treatment effect on interstitial pneumonia and/or pulmonary fibrosis. In addition, a fibrosis suppressive effect of a small-molecule inhibitor of autotaxin in a pulmonary fibrosis model by bleomycin induction has been reported. All these reports show that autotaxin is present in the alveolar lavage fluid of idiopathic pulmonary fibrosis patients, and the concentration and activity thereof are high as compared to healthy individuals.

Idiopathic pulmonary fibrosis is a disease showing extremely poor prognosis as evidenced by a five-year survival rate of 30%. While the mechanism thereof contains many unclear aspects, it is generally understood that damage on alveoli and the like causes excessive action of the tissue repair mechanism, and abnormal growth of fibroblasts and excessive production of connective tissue protein occur in pulmonary interstitium. At present, steroids, immunosuppressants and the like are used for a global standard treatment. In 2008, for the first time in the world, Pirespa (general name: pirfenidone) was approved in Japan as a therapeutic drug for idiopathic pulmonary fibrosis, and the effectiveness thereof and the like are being studied in clinical situations. However, the action mechanism thereof contains many unclear aspects such as what is the target of Pirespa and the like.

Aptamer means a nucleic acid that specifically binds to a target molecule (protein, sugar chain, hormone etc.). It binds to a target molecule due to a three-dimensional structure of a single strand RNA (or DNA). To obtain same, a screening method called a SELEX method (Systematic Evolution of Ligands by Exponential Enrichment) is used. An aptamer obtained by the SELEX method has a chain length of about 80 nucleotides, which is thereafter shortened with a physiological inhibitory activity of the target molecule as an index. It is further modified chemically to improve in vivo stability, thus optimizing same as a pharmaceutical product.

Aptamers show high binding property to the target molecule, and the affinity thereof is often high compared to antibodies having a similar function. Aptamers are unlikely to undergo immune elimination, and adverse reactions characteristic of antibodies, such as antibody-dependent cell-mediated cytotoxicity (ADCC) and complement-dependent cytotoxicity (CDC), do not occur easily with the use of aptamers. From the aspect of delivery, since aptamers are about 1/10 of antibody in molecular size, tissue transfer occurs easily and the delivery of a drug to the object site is easier. Some molecular targeting drugs having a low molecular weight are poorly soluble and require optimization for formulation thereof. Since aptamers have high water-solubility, they are advantageous in such aspect. Furthermore, since aptamers are produced by chemical synthesis, cost-cutting is possible by large-scale production. Besides these, long-term preservation stability and thermal·solvent tolerance are also superior characteristics of the aptamers. On the other hand, the blood half-lives of aptamers are generally shorter than those of antibodies; however, this property is sometimes advantageous in view of toxicity.

In December 2004, the world's first RNA aptamer drug, Macugen, was approved in USA as a therapeutic drug for age-related macular degeneration, and the application of RNA aptamer to a therapeutic drug, a diagnostic agent or a reagent is attracting attention, and the drug is expected to be a next-generation pharmaceutical product.

As aptamer, RNA aptamers have been widely studied. However, since RNA is unstable in vivo and the production cost is high, the research and development of DNA aptamers, which are stable in vivo and can be produced at a low cost, are also ongoing (non-patent documents 1 - 2).

[Document List]

[non-patent documents]

non-patent document 1: Fitzwater and Polisky, Methods Enzymol., 267, 275-301 (1996)
non-patent document 2: Stephen Fitter and Robert James, J. Biol. Chem., 280(40), 34193-34201 (2005)

[SUMMARY OF THE INVENTION]

[Problems to be Solved by the Invention]

The present invention is directed to providing an aptamer for autotaxin and a method of utilizing the same, and the like.

[Means of Solving the Problems]

The present inventors investigated diligently to solve the problem described above and succeeded in preparing an aptamer of good quality for autotaxin, which resulted in the completion of the present invention.
Accordingly, the present invention provides the following invention and the like.

[1] An aptamer that binds to an autotaxin, which comprises a nucleotide sequence represented by the following formula (I):

$$CGGAACC\text{-}N_1\text{-}GGTC \qquad (I)$$

wherein $N_1$ shows any of 3 to 11 nucleotides.
[2] An aptamer that binds to an autotaxin, which comprises a nucleotide sequence represented by the following formula (II):

$$X_3X_1CGGAACC\text{-}N_1\text{-}GGTCX_2X_9 \qquad (II)$$

wherein $N_1$ shows any of 7 to 11 nucleotides, $X_1$, $X_2$, $X_3$ and $X_4$ are each any nucleotide, and at least one of $X_1$ and $X_2$, and $X_3$ and $X_4$ forms Watson-Crick base pairs.
[3] The aptamer of [2], wherein $X_1$ is A, $X_2$ is T, $X_3$ is G, and $X_4$ is C.
[4] An aptamer that binds to an autotaxin, which comprises a nucleotide sequence shown by the following (a), (b) or (c):

(a) a nucleotide sequence selected from SEQ ID NOs: 4 - 14, 16, 20 - 25, 27 and 29;
(b) a nucleotide sequence selected from SEQ ID NOs: 4 - 14, 16, 20 - 25, 27 and 29, wherein one or several nucleotides other than sequences shown by CGGAACC and GGTC in CGGAACC-$N_1$-GGTC wherein $N_1$ shows any of 3 to 11 nucleotides are substituted, deleted, inserted or added; or
(c) a nucleotide sequence having not less than 70% identity with a nucleotide sequence selected from SEQ ID NOs: 4 - 14, 16, 20 - 25, 27 and 29 (excluding sequences shown by CGGAACC and GGTC in CGGAACC-$N_1$-GGTC wherein $N_1$ is as defined above).

[5] The aptamer of any one of [1] - [4], wherein $N_1$ is a nucleotide shown by AGAATACTTTT.
[6] An aptamer that binds to an autotaxin, which has a potential secondary structure represented by the following formula (IV):

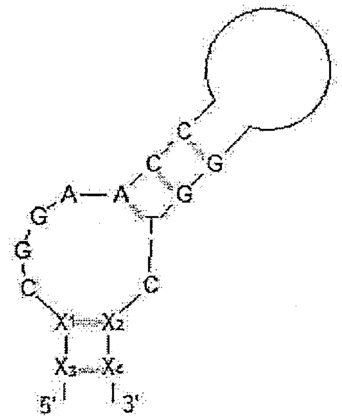


(I V)

wherein $X_1$, $X_2$, $X_3$ and $X_4$ are each any nucleotide, and at least one of $X_1$ and $X_2$, and $X_3$ and $X_4$ forms Watson-Crick base pairs.

[7] The aptamer of [6], wherein $X_1$ is A, $X_2$ is T, $X_3$ is G, and $X_4$ is C.

[8] The aptamer of any one of [1] - [7], which has a base length of not less than 30.

[9] The aptamer of any one of [1] - [8], wherein the hydrogen atoms at the 2'-position of a deoxyribose of respective nucleotides are the same or different and unsubstituted or substituted by an atom or group selected from the group consisting of a fluorine atom and a methoxy group.

[10] The aptamer of any one of [1] - [9], wherein the phosphoric acid groups contained in the aptamer are the same or different and each is unsubstituted or P-alkylated or P-alkoxylated.

[11] An aptamer that binds to an autotaxin, which comprises a nucleotide sequence represented by the following formula (I):

CGGAACC-$N_1$-GGTC (I)

wherein $N_1$ shows any of 3 to 11 nucleotides, and

a hydrophobic group is introduced into a phosphoric acid group between C and C of the nucleotide sequence.

[12] The aptamer of [11], wherein the above-mentioned hydrophobic group is an alkyl group or an alkoxy group.

[13] The aptamer of any one of [1] - [12], wherein at least one nucleotide is modified.

[14] The aptamer of [13], which is modified by inverted dT or polyethylene glycol.

[15] The aptamer of [14], wherein the inverted dT or polyethylene glycol binds to the 5'-terminus or 3'-terminus of the aptamer.

[16] The aptamer of any one of [1] - [15], wherein at least one phosphoric acid group contained in the aptamer is phosphorothioated or phosphorodithioated.

[17] A complex comprising the aptamer of any one of [1] - [16] and a functional substance.

[18] The complex of [17], wherein the functional substance is an affinity substance, a labeling substance, an enzyme, a drug, a toxin or a drug delivery vehicle.

[19] A medicament comprising the aptamer of any one of [1] - [16], or the complex of [17] or [18].

[20] An anti-fibrotic agent comprising the aptamer of any one of [1] - [16], or the complex of [17] or [18].

[21] An autotaxin detection probe, comprising the aptamer of any one of [1] - [16], or the complex of [17] or [18].

[22] A detection method of autotaxin, comprising using the aptamer of any one of [1] - [16], or the complex of [17] or [18].

[Effect of the Invention]

The aptamer and complex of the present invention can be useful as, for example, a medicament or a diagnostic agent or a reagent for various diseases caused by autotaxin such as fibrosis, cancer and the like. The aptamer and complex of the present invention can also be useful for purification and concentration of autotaxin, as well as detection and quantification of autotaxin.

[Brief Description of the Drawings]

Fig. 1 shows (A) secondary structures of aptamers having nucleotide sequences shown in SEQ ID NOs: 4 and 5, and (B) a common secondary structure of a common subsequence represented by the above-mentioned formula (II), predicted by the MFOLD program. In Fig. 1A, the nucleotides of the above-mentioned common subsequence are shown with circled characters.

Fig. 2 shows secondary structures of aptamers having nucleotide sequences shown in SEQ ID NOs: 11 and 12, which are obtained by chain shortening and base substitution of an aptamer having the nucleotide sequence shown in SEQ ID NO: 5, predicted by the MFOLD program. The nucleotides of the common subsequence represented by the above-mentioned formula (II) are shown with circled characters.

Fig. 3 shows binding of an aptamer having a nucleotide sequence shown in SEQ ID NO: 12(48) to an autotaxin. As a capture molecule, autotaxin or FGF2 as a negative control was immobilized, and an aptamer was flown as an analyte. The measurement was performed using Biacore T100 manufactured by GE Healthcare.

Fig. 4 shows the effect of an autotaxin aptamer on collagen accumulation in the lung in bleomycin-induced pulmonary fibrosis model mouse. The left lung was isolated from a group administered with two doses of autotaxin aptamer (SEQ ID NO: 12(48)) every day, and the vehicle administration group, after completion of the administration, hydroxyproline amount per lung weight was measured and compared. The control group is a non-treated (bleomycin non-administrated) mouse.

[Description of Embodiments]

The present invention provides an aptamer having a binding activity to an autotaxin. The aptamer of the present invention can inhibit the activities of autotaxin (phosphodiesterase activity, lysophospholipase D activity etc.).

An aptamer refers to a nucleic acid molecule having a binding activity to a particular target molecule. The aptamer can inhibit the activity of a particular target molecule by binding to the particular target molecule. The aptamer of the present invention has a binding activity to an autotaxin, and can inhibit the activity of autotaxin. The aptamer of the present invention may be a DNA, an RNA, a modified nucleic acid or a mixture thereof. The aptamer of the present invention can also be in a linear or circular form.

Autotaxin (EC.3.1.4.39) is a glycoprotein present in the blood, and is an enzyme that degrades lysophosphatidylcholine (LPC) into lysophosphatidic acid (LPA) and choline. The aptamer of the present invention can exhibit an inhibitory activity against autotaxin derived from any mammals. Such mammals include primates (e.g., human, monkey), rodents (e.g., mouse, rat, guinea pig, hamster), and companion animals, domestic animals and working animals (e.g., dog, cat, horse, bovine, goat, sheep, swine), preferably human.

As for human autotaxin, 4 isotypes of $\alpha$, $\beta$, $\gamma$, $\delta$ have been reported. In the present invention, human autotaxin particularly means $\beta$ type. The amino acid sequence of human $\beta$-autotaxin is identified by accession number NP_001035181, and the human autotaxin also includes a partial protein having a substantially equivalent LPA synthesis activity and a mutated protein wherein a part of the amino acid is substituted, deleted, added or inserted.

The aptamer of the present invention binds to an autotaxin in a physiological buffer. While the buffer is not particularly limited, one having pH about 5.0 - 10.0 is preferably used. Examples of such buffer include below-mentioned solution A (see Example 1). The aptamer of the present invention binds to an autotaxin with the strength of a level detectable by any test shown below.

Biacore T100 manufactured by GE Healthcare is used for the measurement of binding strength. In one measurement method, an aptamer is first immobilized on a sensorchip. The immobilization amount is set to about 1500RU. An autotaxin solution as an analyte prepared to 0.020 $\mu$M is injected by 20 $\mu$L, and binding of the autotaxin to the aptamer is detected. Using DNA containing a random nucleotide sequence consisting of 40 nucleotides as a negative control, when the autotaxin significantly strongly bound to the aptamer as compared to the control DNA, the aptamer can be judged to have a binding ability to autotaxin.

In another measurement method, an autotaxin is first immobilized on a sensorchip. The immobilization amount is set to about 2700RU. An aptamer solution as an analyte prepared to 0.30 $\mu$M is injected by 20 $\mu$L, and binding of the aptamer to the autotaxin is detected. Using DNA containing a random nucleotide sequence consisting of 40 nucleotides as a negative control, when the aptamer significantly strongly bonded to the autotaxin as compared to the control DNA, the aptamer can be judged to have a binding ability to autotaxin.

The inhibitory activity against an autotaxin means an inhibitory ability against any activity that the autotaxin has. While autotaxin has a phosphodiesterase activity to cleave phosphodiester bond by hydrolysis, such activity is inhibited. An acceptable substrate for enzyme activity is not limited to a phosphodiester bond-containing substance (e.g., ATP and the like) present in vivo, and includes a substrate wherein a compound containing same is added with a color development substance or a fluorescent substance. The chromogenic substance and fluorescent substance are known to those of ordinary skill in the art. Also, autotaxin has a lysophospholipase D activity. By this activity, lysophosphatidic acid (LPA) is mainly produced by cleaving the bond on the side opposite from the glycerol backbone of phosphodiester of lysophospholipid. Inhibition of autotaxin activity also includes suppression of the production.

A substrate of autotaxin refers to a substance having a phosphodiester bond to be cleaved by hydrolysis by autotaxin. As a substrate of autotaxin present in vivo, lysophosphatidylcholine (LPC) and sphingosylphosphorylcholine (SPC) are known. The substrate of autotaxin in the present specification also includes LPC and SPC having various carbon chain lengths and degrees of unsaturation, and those added with a chromogenic substance or a fluorescent substance.

Whether an aptamer inhibits the enzyme activity of autotaxin can be evaluated, for example, by the following test. As a substrate of autotaxin, phosphodiester bond-containing synthetic substrate p-nitrophenyl thymidine 5'-monophosphate (pNP-TMP) (SIGMA) is used. A phosphodiester bond is cleaved by hydrolysis, and p-nitrophenol is liberated. The p-nitrophenol develops a yellow color, and the color is detected. For the assay, a 96-well plate (96-Well EIAsurasshuRIA Polystyrene Plates, Costar) was used, and the amount of the reaction mixture was 200 μL. Nucleic acid was prepared in solution A (see below-mentioned Example 1) (100 μL), pNP-TMP (20 μL) adjusted to 10 mM in the reaction mixture A was added, and the mixture was stirred well and heated at 37°C for 5 min. On the other hand, 6 ng of autotaxin (Recombinant Human, manufactured by R&D) diluted with solution A was prepared (80 μL), and heated at 37°C for 5 min. After heating, they were mixed to start an enzyme reaction. The final autotaxin concentration in the reaction solution was 0.3 nM, and the final substrate concentration was 1 mM. A plate containing the reaction mixture was heated at 37°C for 24 hr, placed in a microplate reader SpectraMax190 (manufactured by Molecular Devices) and the absorbance was determined at wavelength 405 nm. The absorbance when nucleic acid is not added as 100% (A0), an enzyme activity rate was determined from the absorbance (A) of each test substance and according to the following formula.

$$\text{Enzyme activity rate} = (A/A0) \times 100$$

The concentration ($IC_{50}$) of an inhibitor necessary for inhibiting the enzyme activity by 50% was determined. An aptamer having an $IC_{50}$ value of not more than 0.10 μM is judged to be an aptamer having a superior inhibitory activity.

The aptamer of the present invention is not particularly limited as long as it binds to any part of an autotaxin. The aptamer is not particularly limited as long as it can inhibit the activity of autotaxin.

The length of the aptamer of the present invention is not particularly limited, and can usually be about 10 to about 200 nucleotides and can be, for example, not more than about 100 nucleotides, preferably not more than about 75 nucleotides. The aptamer of the present invention maintains its activity even with 30 nucleotides. When the total number of nucleotides is smaller, chemical synthesis and mass-production will be easier, and there is a major advantage in terms of cost. It is also thought that chemical modification is easy, stability in the body is high, and toxicity is low. Therefore, the length of the aptamer of the present invention is preferably not less than 30 nucleotides, more preferably not less than 30 nucleotides and not more than 75 nucleotides.

Each nucleotide contained in the aptamer of the present invention is the same or different and can be a nucleotide comprising a hydrogen atom at the 2'-position of deoxyribose (e.g., deoxyribose of pyrimidine nucleotide, deoxyribose of purine nucleotide) (i.e., substituted nucleotide) or a nucleotide wherein hydrogen atom is substituted (modified) by any atom or group at the 2'-position of deoxyribose. Examples of such optional atom or group include a nucleotide substituted by a fluorine atom, a hydroxy group or an alkoxy group (e.g., methoxy group), an acyloxy group (e.g., acetyloxy group), amino group (e.g., $-NH_2$ group), preferably a fluorine atom or a methoxy group.

A preferable example of the aptamer of the present invention is an aptamer that binds to an autotoxin, which comprises a nucleotide sequence represented by the following formula (I):

$$\text{CGGAACC-}N_1\text{-GGTC} \qquad \text{(I)}$$

wherein $N_1$ show any of 3 to 11 nucleotides. The nucleotide number of $N_1$ is preferably 5 - 11, more preferably 7 - 11.

Alternatively, it is an aptamer that binds to an autotoxin, which comprises a nucleotide sequence represented by the following formula (II):

$$X_3X_1\text{CGGAACC-}N_1\text{-GGTC}X_2X_4 \qquad \text{(II)}$$

wherein $N_1$ shows any of 7 to 11 nucleotides, $X_1$, $X_2$, $X_3$ and $X_4$ are each any nucleotide, and at least one of $X_1$ and $X_2$, and $X_3$ and $X_4$ forms Watson-Crick base pairs.

In a preferable one embodiment, at least $X_1$ and $X_2$ form Watson-Crick base pairs, more preferably, both $X_1$ and $X_2$, and $X_3$ and $X_4$ form Watson-Crick base pairs. When $X_1$ and $X_2$ form Watson-Crick base pairs, ($X_1$-$X_2$) is preferably (A-T), (C-G) or (T-A). When $X_3$ and $X_4$ form Watson-Crick base pairs, ($X_3$-$X_4$) is preferably (G-C) or (C-G). When both $X_1$ and $X_2$, and $X_3$ and $X_4$ form Watson-Crick base pairs, ($X_1$-$X_2$, $X_3$-$X_4$) is preferably (A-T, G-C), (C-G, G-C) or (T-A, C-G), more preferably (A-T, G-C).

Alternatively, the aptamer of the present invention may be an aptamer that binds to an autotoxin, which comprises a nucleotide sequence shown by the following (a), (b) or (c):

(a) a nucleotide sequence selected from SEQ ID NOs: 4 - 14, 16, 20 - 25, 27 and 29;
(b) a nucleotide sequence selected from SEQ ID NOs: 4 - 14, 16, 20 - 25, 27 and 29, wherein one or several nucleotides other than sequences shown by CGGAACC and GGTC in CGGAACC-$N_1$-GGTC wherein $N_1$ shows any of 3 to 11 nucleotides are substituted, deleted, inserted or added; or
(c) a nucleotide sequence having not less than 70% identity with a nucleotide sequence selected from SEQ ID NOs: 4 - 14, 16, 20 - 25, 27 and 29 (provided that sequences shown by CGGAACC and GGTC in CGGAACC-$N_1$-GGTC wherein $N_1$ is as defined above are the same).

The aptamer of the above-mentioned (b) or (c) binds to an autotaxin. The aptamer can inhibit activity of autotaxin (enzyme activity etc. of autotaxin).
In the above-mentioned (b), the number of nucleotides to be substituted, deleted, inserted or added is not particularly limited as long as the aptamer can bind to an autotaxin and/or inhibit activity of autotaxin (enzyme activity etc. of autotaxin). For example, it may be not more than about 30, preferably not more than about 20, more preferably not more than about 10, further preferably not more than 5, most preferably 4, 3, 2 or 1. The substitution here also includes base substitution from T (thymine) to U (uracil) (substitution from thymidine to deoxyuridine as nucleotides).
In the above-mentioned (c), the "identity" means a ratio (%) of the same nucleotide residues to all overlapping nucleotide residues in an optimal alignment (preferably, the algorithm can take into consideration an introduction of a gap into one or both of the sequences for optimal alignment) when two nucleotide sequences are aligned using a mathematical algorithm known in the art.
In the present specification, the identity of nucleotide sequence can be calculated by, for example, aligning two nucleotide sequences under the following conditions (gap opening =5 penalties; gap extension =2 penalties; x_drop off =50; expectancy =10; filtering=ON) by using homology calculation algorithm NCBI BLAST-2 (National Center for Biotechnology Information Basic Local Alignment Search Tool).
The aptamer of the present invention can also be

(d) a conjugate of a plurality of one or more of the above-mentioned (a) and/or one or more of the above-mentioned (b) and/or one or more of the above-mentioned (c). Here, conjugation can be achieved by tandem binding. In the conjugation, a linker may be utilized. As the linker, nucleotide chains (e.g., 1 to about 20 nucleotides) and non-nucleotide chains (e.g., $-(CH_2)_n-$ linker, $-(CH_2CH_2O)_n-$ linker, hexaethylene glycol linker, TEG linker, peptide-containing linker, -S-S- bond-containing linker, -CONH- bond-containing linker, $-OPO_3-$ bond-containing linker) can be mentioned. The plurality as mentioned in the above-described conjugate of a plurality thereof is not particularly limited, as long as it is two or more, and the plurality can be, for example, 2, 3 or 4.

The nucleotides in the above-mentioned (a) - (d) are the same or different and each may be a deoxyribonucleotide wherein the 2'-position of deoxyribose is a hydrogen atom, or a nucleotide wherein a hydrogen atom at the 2'-position of deoxyribose is substituted by any atom or group (e.g., fluorine atom, hydroxy group or methoxy group).
In one preferable embodiment, $N_1$ in the above-mentioned formula (I) or (II) is represented by the following formula (III):

$$X_5X_7X_9\text{-}N_2\text{-}X_{10}X_8X_6 \qquad \text{(III)}$$

wherein $N_2$ is any of 1 - 5 nucleotides, $X_5$ - $X_{10}$ are each any nucleotide, at least one of $X_5$ and $X_6$, $X_7$ and $X_8$, and $X_9$ and $X_{10}$ form Watson-Crick base pairs, and $X_5$ and $X_6$ form Watson-Crick base pairs or G:T base pairs). Preferably, when $X_5$ and $X_6$ form Watson-Crick base pairs, ($X_5$-$X_6$) is (A-T), when G:T base pairs is formed, ($X_5$-$X_6$) is (G:T). When $X_5$ and $X_6$ form G:T base pairs, $X_7$ and $X_8$ preferably form Watson-Crick base pairs. On the other hand, when $X_5$ and $X_6$ form Watson-Crick base pairs, at least one of $X_7$ and $X_8$, and $X_9$ and $X_{10}$ preferably form Watson-Crick base pairs. When $X_7$ and $X_8$ form G:T base pairs, $X_9$ and $X_{10}$ preferably form Watson-Crick base pairs.
The nucleotide number of $N_2$ is preferably 3 - 5.
In a particularly preferable embodiment, $N_1$ in the formula (I) or (II) is AGAATACTTTT (SEQ ID NO: 30).
The sequence shown by the above-mentioned formula (II):

$$X_3X_1\text{CGGAACC-}N_1\text{-GGTC}X_2X_4 \qquad \text{(II)}$$

wherein $N_1$, $X_1$, $X_2$, $X_3$ and $X_4$ are as defined above can form a potential secondary structure shown by the formula (IV):

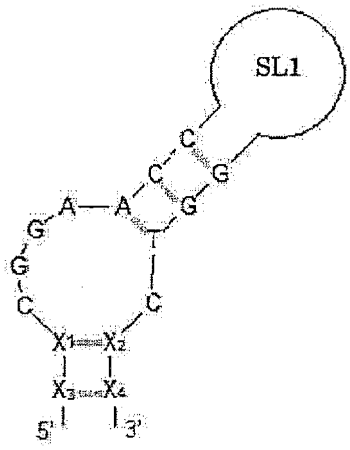


(I V)

The aptamer of the present invention is considered to show various activities since it has the above-mentioned structure in the subsequence shown by the above-mentioned formula (II).

In addition, a stem structure can be preferably formed by an interaction between the sequences following the 5'-terminus and 3'-terminus in the above-mentioned structure. Particularly, at least one of $X_1$ and $X_2$, and $X_3$ and $X_4$ forms Watson-Crick base pairs.

For the aptamer of the present invention to show various activities, a stem-loop structure shown by the above-mentioned potential secondary structure is desirably maintained. While a stem structure can be formed by complementary base pairs, the number of base pairs is not particularly limited. In the stem structure, even when base pairs are not formed in a part thereof, the aptamer activity is maintained as long as a stem structure is constituted as a whole.

The stem-loop part SL1 in the upper right of the formula (IV) corresponds to $N_1$ part of the formula (II). When $N_1$ is a sequence represented by the above-mentioned formula (III), the stem-loop structure represented by the formula (IV) is considered to be more stably maintained. That is, $X_5$ - $X_{10}$ in the formula (III) form a stem structure of SL1 part of the formula (IV).

In the aptamer of the present invention, at least one kind (e.g., 1, 2, 3 or 4 kinds) of nucleotide can also be a nucleotide comprising a hydrogen atom, or the above-described any atom or group, for example, at least two kinds (e.g., 2, 3 or 4 kinds) of atoms or groups selected from the group consisting of a fluorine atom, a hydroxy group and a methoxy group, at the 2'-position of deoxyribose.

The aptamer of the present invention can also be a nucleotide wherein all nucleotides have a hydrogen atom, or the aforementioned any atom or group, for example, the same group selected from the group consisting of a fluorine atom, a hydroxy group and a methoxy group, at the 2'-position of deoxyribose.

In this Description, the nucleotides constituting the aptamer are assumed to be DNAs (i.e., the sugar groups are assumed to be deoxyribose) in describing how the sugar groups are modified in the nucleotides. However, this does not mean that RNA is completely exempted from the aptamer-constituting nucleotides, and a modification should read as a modification of RNA as appropriate. When the nucleotide constituting the aptamer is RNA, for example, substitution of a hydrogen atom at the 2'-position of deoxyribose by X should read as a substitution of the hydroxy group at the 2'-position of ribose by X.

The aptamer of the present invention may be one wherein a sugar residue (e.g., deoxyribose) of each nucleotide has been modified to increase the autotaxin binding activity, stability, drug deliverability and the like. As examples of the modification in a sugar residue, substitution of hydrogen atom at the 2'-position, or a hydroxy group at the 3'-position and/or 4'-position of the sugar residue with another atom, and the like can be mentioned. As the kind of the modification, fluorination, alkoxylation (e.g., methoxylation, ethoxylation), 0-arylation, S-alkylation (e.g., S-methylation, S-ethylation), S-arylation, and amination (e.g., $-NH_2$) can be mentioned. Such alterations in the sugar residue can be performed by a method known per se (see, for example, Sproat et al., (1991) Nucl. Acid. Res. 19, 733-738; Cotton et al., (1991) Nucl. Acid. Res. 19, 2629-2635; Hobbs et al., (1973) Biochemistry 12, 5138-5145).

The sugar residue may also be BNA: Bridged nucleic acid (LNA: Linked nucleic acid), wherein a crosslinking structure is formed at the 2'-position and the 4'-position. Such alteration of the sugar residue can also be performed by a method known per se (e.g., Tetrahedron Lett., 38, 8735-8738 (1997); Tetrahedron, 59, 5123-5128 (2003), Rahman S.M.A., Seki S., Obika S., Yoshikawa H., Miyashita K., Imanishi T., J. Am. Chem. Soc., 130, 4886-4896 (2008) and the like).

The aptamer of the present invention may also have a nucleic acid base (e.g., purine or pyrimidine) altered (e.g., chemical substitution) to increase the autotaxin binding activity and the like. As examples of such alterations,

pyrimidine alteration at 5-position, purine alteration at 6- and/or 8-position(s), alteration with an extracyclic amine, substitution with 4-thiouridine, and substitution with 5-bromo or 5-iodo-uracil can be mentioned.

In addition, the phosphate group contained in the aptamer of the present invention may be altered to confer resistance to nuclease and hydrolysis, increase the autotaxin binding activity and the like. For example, the P(O)O group as a phosphoric acid group may be substituted with P(O)S (thioate), P(S)S (dithioate), P(O)$NR_2$ (amidate), P(O)R, P(O)OR', CO or $CH_2$ (formacetal) or 3'-amine (-NH-$CH_2$-$CH_2$-) [wherein each unit of R or R' is independently H or a substituted or unsubstituted alkyl (e.g., methyl, ethyl)].

Of these, one wherein at least one of the P(O)O groups to be the phosphoric acid group is substituted by P(O)S (thioate), P(S)S (dithioate) or P(O)R, P(O)OR' (R and R' are unsubstituted alkyl groups), i.e., phosphorothioated, phosphorodithioated, P-alkylated or P-alkoxylated, is preferable. When at least one of the phosphoric acid groups contained in the aptamer is phosphorothioated, phosphorodithioated, P-alkylated or P-alkoxylated, the activity of the aptamer of the present invention is improved.

Here, particularly a P-methylated or P-alkoxylated phosphoric acid group is preferably introduced into a part of the common sequence. Introduction of such functional group having high hydrophobicity (hereinafter to be indicated as "hydrophobic group") into the common subsequence is effective for improving the inhibitory activity of the aptamer of the present invention by a direct action with autotaxin. While the hydrophobic group is not particularly limited as long as the inhibitory activity of the aptamer of the present invention against autotaxin is improved, an alkyl group or an alkoxy group is preferable. Examples of the alkyl group include methyl group, ethyl group, propyl group and the like, and examples of the alkoxy group include methoxy group, ethoxy group, isopropoxy group, butoxy group, propoxy group and the like.

Particularly, in the present invention, a nucleotide sequence represented by the following formula (I):

CGGAACC-$N_1$-GGTC (I)

wherein $N_1$ show any of 3 to 11 nucleotides, or a nucleotide sequence represented by the following formula (II):

$X_3X_1$CGGAACC-$N_1$-GGTC$X_2X_4$ (II)

wherein $N_1$ shows any of 7 to 11 nucleotides, $X_1$, $X_2$, $X_3$ and $X_4$ are each any nucleotide, and at least one of $X_1$ and $X_2$, and $X_3$ and $X_4$ forms Watson-Crick base pairs, or a nucleotide sequence wherein a hydrophobic group is introduced into a phosphoric acid group between C and C in a potential secondary structure shown by the formula (IV):

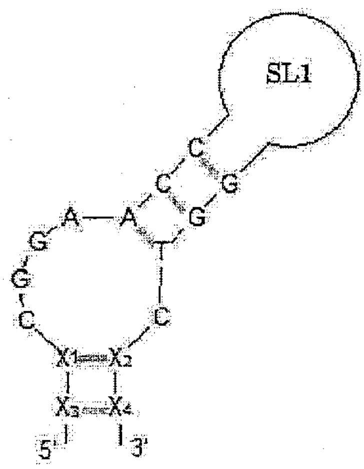


(IV)

is preferably used, and an aptamer containing such sequence is preferable as the aptamer of the present invention. Introduction of such hydrophobic group into the common subsequence is effective for improving the inhibitory activity of the aptamer of the present invention by a direct action with autotaxin. While the hydrophobic group is not particularly limited as long as the inhibitory activity of the aptamer of the present invention against autotaxin is improved, an alkyl group or an alkoxy group is preferable. Specific groups are as indicated above as examples.

The linking group is, for example, -O-, -N- or -S-, and nucleotides can bind to an adjoining nucleotide via these linking groups.

The alterations may also include alterations such as capping at 3' and 5'.

An alteration can further be performed by adding to an end a polyethyleneglycol, amino acid, peptide, inverted dT, nucleic acid, nucleosides, myristoyl, lithocolic-oleyl, docosanyl, lauroyl, stearoyl, palmitoyl, oleoyl, linoleoyl, other lipids, steroids, cholesterol, caffeine, vitamins, dyes, fluorescent substances, anticancer agents, toxins, enzymes, radioactive substances, biotin and the like. For such alterations, see, for example, US Patents 5,660,985 and 5,756,703.

The aptamer of the present invention can be chemically synthesized as disclosed herein and by a method known per se in the art. For example, it can be synthesized using DNA polymerase. DNA having an object sequence is chemically synthesized and, using same as a template, amplification is performed by a known method of polymerase chain reaction (PCR). This is converted to a single strand by an already-known method of polyacrylamide electrophoresis, enzyme treatment method such as λ exonuclease and the like, a method using streptavidin-biotin interaction and alkali treatment and the like. When a modified aptamer is synthesized, the efficiency of elongation reaction can be increased by using a polymerase introduced with a mutation into a specific site. The thus-obtained aptamer can be purified easily by a known method.

Aptamer can be synthesized in a large amount by a chemical synthesis method such as amidite method, phosphoramidite method and the like. The synthesis method is a well-known method, and as described in Nucleic Acid (Vol. 2) [1] Synthesis and Analysis of Nucleic Acid (Editor: Yukio Sugiura, Hirokawa Publishing Company) and the like. In fact, a synthesizer such as OligoPilot100, OligoProcess and the like manufactured by GE Healthcare Bioscience is used. Purification is performed by a method known per se such as chromatography and the like.

A functional substance can be added to the aptamer after synthesis by introducing active groups such as amino group and the like during chemical synthesis by the phosphoramidite method and the like. For example, a polyethylene glycol chain introduced with a carboxyl group can be condensed by introducing an amino group into the terminal of the aptamer.

An aptamer binds to the target substance in a wide variety of binding modes, such as ionic bonds based on the negative charge of the phosphate group, hydrophobic bonds and hydrogen bonds based on ribose, and hydrogen bonds and stacking interaction based on nucleic acid bases. In particular, ionic bonds based on the negative charge of the phosphate group, which are present in the same number as the number of constituent nucleotides, are strong, and bind to lysine and arginine being present on the surface of the positive charge of protein. For this reason, nucleic acid bases not involved in the direct binding to the target substance can be substituted. In particular, because the region of stem structure has already formed base pairs and faces the inside of the double helical structure, nucleic acid bases are unlikely to bind directly to the target substance. Therefore, even when a base pair is substituted with another base pair, the activity of the aptamer often does not decrease. In structures wherein no base pairs are formed, such as loop structures, provided that the nucleic acid base is not involved in the direct binding to the target molecule, base substitution is possible. Regarding modifications of the 2'-position of deoxyribose, the functional group at the 2'-position of deoxyribose infrequently interacts directly with the target molecule, but in many cases, it is of no relevance, and can be substituted by another modified molecule. Hence, an aptamer, unless the functional group involved in the direct binding to the target molecule is substituted or deleted, often retains the activity thereof. It is also important that the overall three-dimensional structure does not change substantially.

An aptamer can be prepared by utilizing DNA-SELEX method and an improved method thereof (e.g., Stephen Fitter and Robert James, J. Biol. Chem., 280(40), 34193-34201 (2005) etc.). In the SELEX method, by setting strict selection conditions by increasing the number of rounds or using a competing substance, an aptamer exhibiting a stronger binding potential for the target substance is concentrated and selected. Hence, by adjusting the number of rounds of SELEX and/or changing the competitive condition, aptamers with different binding forces, aptamers with different binding modes, and aptamers with the same binding force or binding mode but different base sequences can be obtained in some cases. The SELEX method comprises a process of amplification by PCR; by causing a mutation by using manganese ions and the like in the process, it is possible to perform SELEX with higher diversity.

The aptamers obtained by SELEX are nucleic acids that exhibit high affinity for the target substance, but this does not mean inhibiting the physiological activity of the target substance. Autotaxin is a basic protein, and is considered to be likely to allow nucleic acids to bind thereto nonspecifically. An aptamer that does not bind to a specific site does not influence the activity of the target substance. In fact, the RNA containing a random sequence that was used as a negative control did not bind to or inhibit autotaxin.

Based on the active aptamer thus selected, SELEX can be performed using a different primer in an attempt to obtain an aptamer having a higher activity. As a specific method, SELEX is performed again after preparing a template wherein an aptamer with a determined sequence is partially randomized or a template doped with about 10 to 30% of random sequences.

An aptamer obtained by SELEX has a length of about 80 nucleotides, and this is difficult to prepare as a pharmaceutical as it is. Hence, it is necessary to repeat try-and-error efforts to shorten the aptamer to a length permitting easy chemical synthesis, which is 50 nucleotides or less. Depending on the primer design for an aptamer obtained by SELEX, the ease of the subsequent minimization operation changes. Unless the primer is designed successfully, subsequent development will be impossible even if an aptamer with activity is selected by SELEX. In the present invention,

an aptamer maintaining an inhibitory activity could be obtained even with about 30 nucleotides.

Aptamers are altered easily since they permit chemical synthesis. For aptamers, by predicting the secondary structure using the MFOLD program, or by predicting the steric structure by X-ray analysis or NMR analysis, it is possible to predict to some extent which nucleotide can be substituted or deleted, where to insert a new nucleotide and the like. A predicted aptamer with the new sequence can easily be chemically synthesized, and it can be determined whether or not the aptamer retains the activity using an existing assay system.

When a region important to the binding of the obtained aptamer with the target substance is identified by repeated try-and-error efforts as described above, the activity remains unchanged in many cases even when a new sequence is added to both ends of the sequence. The length of the new sequence is not particularly limited.

Modifications, like sequences, permitting a wide range of design or alterations, can be freely performed by those of ordinary skill in the art.

As stated above, aptamers permit a wide range of design or alterations. The present invention also provides a production method of aptamer that enables a wide range of design or alteration of an aptamer comprising a specified sequence (e.g., a sequence corresponding to a portion selected from among stem regions, internal loop regions, bulge regions, hairpin loop regions and single-strand regions: hereinafter, abbreviated as fixed sequence as required).

For example, such production method of aptamer includes production of an aptamer comprising a fixed sequence by using a single kind of nucleic acid molecule consisting of a nucleotide sequence shown by:

sequence for primer (i) -(N)a-fixed sequence-(N)b- sequence for primer (ii)

wherein (N)a represents a nucleotide chain consisting of "a" units of N; (N)b represents a nucleotide chain consisting of "b" units of N; each of the units of N, whether identical or different, is a nucleotide selected from the group consisting of A, G, C, U and T (preferably, A, G, C and T). Each of "a" and "b", whether identical or different, can be any numbers, and can be, for example, 1 to about 100, preferably 1 to about 50, more preferably 1 to about 30, still more preferably 1 to about 20 or 1 to about 10], or plural kinds of nucleic acid molecules (e.g., library of nucleic acid molecule different in the number of a, b etc.) and primer pairs corresponding to the sequences for primer (i) and (ii), respectively.

The present invention also provides a complex comprising the aptamer of the present invention and a functional substance bound thereto. The binding between the aptamer and the functional substance in the complex of the present invention can be a covalent bond or a non-covalent bond. The complex of the present invention can be one wherein the aptamer of the present invention and one or more (e.g., 2 or 3) of functional substances of the same kind or different kinds are bound together. The functional substance is not particularly limited, as far as it newly confers a certain function to an aptamer of the present invention, or is capable of changing (e.g., improving) a certain characteristic which an aptamer of the present invention can possess. As examples of the functional substance, proteins, peptides, amino acids, lipids, sugars, monosaccharides, polynucleotides, and nucleotides can be mentioned. As examples of the functional substance, affinity substances (e.g., biotin, streptavidin, polynucleotides possessing affinity for target complementary sequence, antibodies, glutathione Sepharose, histidine), substances for labeling (e.g., fluorescent substances, luminescent substances, radioisotopes), enzymes (e.g., horseradish peroxidase, alkaline phosphatase), drug delivery vehicles (e.g., liposome, microspheres, peptides, polyethyleneglycols), drugs (e.g., those used in missile therapy such as calicheamycin and duocarmycin; nitrogen mustard analogues such as cyclophosphamide, melphalan, ifosfamide or trofosfamide; ethylenimines such as thiotepa; nitrosoureas such as carmustine; alkylating agents such as temozolomide or dacarbazine; folate-like metabolic antagonists such as methotrexate or raltitrexed; purine analogues such as thioguanine, cladribine or fludarabine; pyrimidine analogues such as fluorouracil, tegafur or gemcitabine; vinca alkaloids such as vinblastine, vincristine or vinorelbine and analogues thereof; podophyllotoxin derivatives such as etoposide, taxans, docetaxel or paclitaxel; anthracyclines such as doxorubicin, epirubicin, idarubicin and mitoxantrone, and analogues thereof; other cytotoxic antibiotics such as bleomycin and mitomycin; platinum compounds such as cisplatin, carboplatin and oxaliplatin; pentostatin, miltefosine, estramustine, topotecan, irinotecan and bicalutamide), and toxins (e.g., ricin toxin, liatoxin and vero toxin) can be mentioned. These functional molecules are finally removed in some cases. Furthermore, the molecules may be peptides that can be recognized and cleaved by enzymes such as thrombin, matrix metalloproteinase (MMP), and Factor X, and may be polynucleotides that can be cleaved by nucleases or restriction endonuclease.

The aptamer and the complex of the present invention can be used as, for example, a medicament, a diagnostic reagent, a test reagent or a reagent.

The aptamer and complex of the present invention can have an activity to inhibit the function of autotaxin. As mentioned above, autotaxin is deeply involved in the fibrosis of organ or tissue. Therefore, the aptamer and complex of the present invention is useful as a medicament for the treatment or prophylaxis of diseases involving organ or tissue

fibrosis, particularly diseases associated with fibrosis of various tissues.

Here, examples of the diseases involving organ or tissue fibrosis include pulmonary fibrosis, prostatic hyperplasia, fibrosis of myocardium, myocardial fibrosis, musculoskeletal fibrosis, bone-marrow fibrosis, hysteromyoma, scleroderma, post-surgical adhesion, post-surgical scar, burn scar, hypertrophic scar, keloid, atopic dermatitis, peritoneal sclerosis, asthma, cirrhosis, chronic pancreatitis, scirrhous stomach cancer, hepatic fibrosis, renal fibrosis, fibrous vascular disease, retinopathy due to fibrous microvasculitis as complication of diabetes, neurosis, nephropathy, glomerulonephritis, renal tubule interstitial nephritis, hereditaryrenal diseases, arteriosclerosis peripheral arteritis and the like.

Autotaxin has an enzyme activity, and cleaves a physiologically active substance to be the substrate thereof. LPA is mainly produced from LPC, and LPA binds to a receptor thereof expressed on a cellular surface, activates intracellular G protein, and further, PLC, ERK and Rho at the downstream thereof, and exhibits physiological actions such as cell proliferation, survival, and migration. Therefore, the aptamer and complex of the present invention can be used as medicaments, diagnostic agents, test drugs, or reagents for diseases relating to activation of these pathways. As the disease, the above-mentioned diseases involving organ or tissue fibrosis can be mentioned.

The medicament of the present invention can be one formulated with a pharmaceutically acceptable carrier. As examples of the pharmaceutically acceptable carrier, excipients such as sucrose, starch, mannit, sorbit, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrants such as starch, carboxymethylcellulose, hydroxypropylstarch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizin-ammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like can be mentioned, but these are not limitative.

While the administration route of the medicament of the present invention is not particularly limited, for example, oral administration and parenteral administration can be mentioned. Preparations suitable for oral administration are a solution prepared by dissolving an effective amount of ligand in a diluent such as water, physiological saline, or orange juice; capsules, sachets or tablets comprising an effective amount of ligand in solid or granular form; a suspension prepared by suspending an effective amount of active ingredient in an appropriate dispersant; an emulsion prepared by dispersing and emulsifying a solution of an effective amount of active ingredient in an appropriate dispersant, and the like.

The medicament of the present invention can be coated by a method known per se for the purpose of taste masking, enteric dissolution, sustained release and the like as necessary. As examples of coating agents used for the coating, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (manufactured by Rohm, Germany, methacrylic acid/acrylic acid copolymer), dyes (e.g., red iron oxide, titanium dioxide and the like) and the like are used. The medicament may be a rapid-release preparation or sustained-release preparation. Examples of the base of the sustained release include liposome, atelocollagen, gelatin, hydroxyapatite, PLGA and the like.

As preparations suitable for parenteral administration (e.g., intravenous administration, subcutaneous administration, intramuscular administration, topical administration, intraperitoneal administration, intranasal administration, pulmonary administration and the like), aqueous and non-aqueous isotonic sterile injectable liquids are available, which may comprise an antioxidant, a buffer solution, a bacteriostatic agent, an isotonizing agent and the like. Aqueous and non-aqueous sterile suspensions can also be mentioned, which may comprise a suspending agent, a solubilizer, a thickener, a stabilizer, an antiseptic and the like. The preparation can be included in a container such as an ampoule or a vial in a unit dosage volume or in several divided doses. An active ingredient and a pharmaceutically acceptable carrier can also be freeze-dried and stored in a state that may be dissolved or suspended in an appropriate sterile vehicle just before use. Sustained-release preparations are also suitable preparations. The sustained-release preparations include sustained release from carriers or containers embedded in the body, such as artificial bones, biodegradable or non-degradable sponges, bags, drug pumps, osmotic pressure pumps and the like. Devices for continuous or intermittent, systemic or topical delivery from outside the body are also included in the scope of sustained-release preparations. Biodegradable bases include liposome, cationic liposome, poly(lactic-co-glycolic) acid (PLGA), atelocollagen, gelatin, hydroxyapatite, polysaccharide sizofiran. In addition to liquid injections and sustained-release preparations, inhalants and ointments are also acceptable. In the case of an inhalant, an active ingredient in a freeze-dried state is micronized and administered by inhalation using an appropriate inhalation device. An inhalant can be formulated as appropriate with a conventionally used surfactant, oil, seasoning, cyclodextrin or derivative thereof and the like as required.

Here, as examples of the surfactant, oleic acid, lecithin, diethylene glycol dioleate, tetrahydroflufuryl oleate, ethyl oleate, isopropyl myristate, glyceryl trioleate, glyceryl monolaurate, glyceryl monooleate, glyceryl monostearate, glyceryl monolysinoate, cetyl alcohol, stearyl alcohol, polyethyleneglycol 400, cetylpyridinium chloride, sorbitan trioleate

(trade name, Span 85), sorbitan monoleate (trade name, Span 80), sorbitan monolaurate (trade name, Span 20), polyoxyethylene hardened castor oil (trade name, HCO-60), polyoxyethylene (20) sorbitan monolaurate (trade name, Tween 20), polyoxyethylene (20) sorbitan monooleate (trade name, Tween 80), lecithin of natural resource origin (trade name, Epiclon), oleylpolyoxyethylene (2) ether (trade name, Brij 92), stearyl polyoxyethylene (2) ether (trade name, Brij 72), lauryl polyoxyethylene (4) ether (trade name, Brij 30), oleylpolyoxyethylene (2) ether (trade name, Genapol 0-020), block copolymer of oxyethylene and oxypropylene (trade name, Synperonic) and the like can be mentioned. As examples of the oil, corn oil, olive oil, cottonseed oil, sunflower oil and the like can be mentioned. In the case of an ointment, an appropriate pharmaceutically acceptable base (yellow petrolatum, white petrolatum, paraffin, plastibase, silicone, white ointment, beeswax, lard, vegetable oils, hydrophilic ointment, hydrophilic petrolatum, purified lanolin, hydrolyzed lanolin, water-absorbing ointment, hydrophilic plastibase, macrogol ointment and the like) is blended with an active ingredient, and used as a preparation.

An inhalant can be produced according to a conventional method. Specifically, an inhalant can be produced by powdering or liquefying the above-described aptamer and complex of the present invention, blending it in an inhalation propellant and/or carrier, and filling them in an appropriate inhalation vessel. When the above-described aptamer and complex of the present invention is a powder, an ordinary mechanical powder inhalator can be used; in the case of a liquid, an inhalator such as a nebulizer can be used. Here, as the propellant, conventionally known one can be widely used; chlorofluorocarbon-series compounds such as chlorofluorocarbon-11, chlorofluorocarbon-12, chlorofluorocarbon-21, chlorofluorocarbon-22, chlorofluorocarbon-113, chlorofluorocarbon-114, chlorofluorocarbon-123, chlorofluorocarbon-142c, chlorofluorocarbon-134a, chlorofluorocarbon-227, chlorofluorocarbon-C318, and 1,1,1,2-tetrafluoroethane, hydrocarbons such as propane, isobutane, and n-butane, ethers such as diethyl ether, compressed gases such as nitrogen gas and carbon dioxide gas and the like can be mentioned.

As examples of the surfactant, oleic acid, lecithin, diethylene glycol dioleate, tetrahydroflufuryl oleate, ethyl oleate, isopropyl myristate, glyceryl trioleate, glyceryl monolaurate, glyceryl monooleate, glyceryl monostearate, glyceryl monolysinoate, cetyl alcohol, stearyl alcohol, polyethyleneglycol 400, cetylpyridinium chloride, sorbitan trioleate (trade name, Span 85), sorbitan monoleate (trade name, Span 80), sorbitan monolaurate (trade name, Span 20), polyoxyethylene hardened castor oil (trade name, HCO-60), polyoxyethylene (20) sorbitan monolaurate (trade name, Tween 20), polyoxyethylene (20) sorbitan monooleate (trade name, Tween 80), lecithin of natural resource origin (trade name, Epiclon), oleylpolyoxyethylene (2) ether (trade name, Brij 92), stearyl polyoxyethylene (2) ether (trade name, Brij 72), lauryl polyoxyethylene (4) ether (trade name, Brij 30), oleylpolyoxyethylene (2) ether (trade name, Genapol 0-020), block copolymer of oxyethylene and oxypropylene (trade name, Synperonic) and the like can be mentioned. As examples of the oil, corn oil, olive oil, cottonseed oil, sunflower oil and the like can be mentioned. In the case of an ointment, an appropriate pharmaceutically acceptable base (yellow petrolatum, white petrolatum, paraffin, plastibase, silicone, white ointment, beeswax, lard, vegetable oils, hydrophilic ointment, hydrophilic petrolatum, purified lanolin, hydrolyzed lanolin, water-absorbing ointment, hydrophilic plastibase, macrogol ointment and the like) is blended with the aptamer of the present invention as an active ingredient, and used as a preparation.

The dosage of the medicament of the present invention varies depending on the kind and activity of active ingredient, seriousness of disease, animal species being the subject of administration, drug tolerability of the subject of administration, body weight, age and the like, and the usual dosage, based on the amount of active ingredient per day for an adult, can be about 0.0001 to about 100 mg/kg, for example, about 0.0001 to about 10 mg/kg, preferably about 0.005 to about 1 mg/kg.

The aptamer and complex of the present invention can specifically bind to an autotaxin. Therefore, the aptamer and complex of the present invention is useful as a probe for autotaxin detection. The probe is useful for in vivo imaging, blood concentration measurement, tissue staining, ELISA and the like of autotaxin. In addition, the probe is useful as a diagnostic agent, test drug, reagent and the like for diseases involving autotaxin (fibrosis, disease accompanied by malignant tumor etc.).

Also, based on the specific binding to an autotaxin, the aptamer and complex of the present invention can be used as a ligand for autotaxin separation and purification.

In addition, the aptamer and complex of the present invention can be used as a drug delivery agent to a part where autotaxin is localized in vivo.

The present invention also provides a solid phase carrier having the aptamer and the complex of the present invention immobilized thereon. As examples of the solid phase carrier, a substrate, a resin, a plate (e.g., multiwell plate), a filter, a cartridge, a column, and a porous material can be mentioned. The substrate can be one used in DNA chips, protein chips and the like; for example, nickel-PTFE (polytetrafluoroethylene) substrates, glass substrates, apatite substrates, silicon substrates, alumina substrates and the like, and substrates prepared by coating these substrates with a polymer and the like can be mentioned. As examples of the resin, agarose particles, silica particles, a copolymer of acrylamide and N,N'-methylenebisacrylamide, polystyrene-crosslinked divinylbenzene particles, particles of dextran crosslinked with epichlorohydrin, cellulose fiber, crosslinked polymers of aryldextran and N,N'-methylenebisacrylamide, monodispersed synthetic polymers, monodispersed hydrophilic polymers, Sepharose, Toyopearl and the like can be

mentioned, and also resins prepared by binding various functional groups to these resins were included. The solid phase carrier of the present invention can be useful in, for example, purifying, detecting and quantifying autotaxin.

The aptamer and the complex of the present invention can be immobilized onto a solid phase carrier by a method known per se. For example, a method that introduces an affinity substance (e.g., those described above) or a predetermined functional group into the aptamer or the complex of the present invention, and then immobilizes the aptamer and complex onto a solid phase carrier via the affinity substance or predetermined functional group can be mentioned. The present invention also provides such methods. The predetermined functional group can be a functional group that can be subjected to a coupling reaction; for example, an amino group, a thiol group, a hydroxy group, and a carboxyl group can be mentioned. The present invention also provides an aptamer having such a functional group introduced thereinto.

The present invention also provides a method of purifying and concentrating autotaxin. In particular, the present invention makes it possible to separate autotaxin from other family proteins. The method of purification and concentration of the present invention can comprise adsorbing autotaxin to the solid phase carrier of the present invention, and eluting the adsorbed autotaxin with an eluent. Adsorption of autotaxin to the solid phase carrier of the present invention can be achieved by a method known per se. For example, an autotaxin-containing sample (e.g., bacterial or cell culture or culture supernatant, blood) is introduced into the solid phase carrier of the present invention or a composition containing the same. Autotaxin can be eluted using an eluent such as a neutral solution. There is no limitation on the neutral eluent, which can have a pH of, for example, about 6 to about 9, preferably about 6.5 to about 8.5, and more preferably about 7 to about 8. The neutral solution can also comprise, for example, urea, chelating agent (e.g., EDTA), sodium salt (e.g., NaCl), a potassium salt (e.g., KCl), a magnesium salt (e.g., $MgCl_2$), a surfactant (e.g., Tween 20, Triton, NP40), and glycerin. The method of purification and concentration of the present invention can further comprise washing the solid phase carrier using a washing solution after autotaxin adsorption. Examples of the washing solution include those containing urea, a chelating agent (e.g., EDTA), Tris, an acid, an alkali, Transfer RNA, DNA, surfactants such as Tween 20, salts such as NaCl and the like. The method of purification and concentration of the present invention can still further comprise heating the solid phase carrier. This step enables the regeneration and sterilization of the solid phase carrier.

The present invention also provides a method of detecting and quantifying autotaxin. In particular, the present invention makes it possible to detect and quantify autotaxin separately from the proteins of other family proteins. The method of detection and quantitation of the present invention can comprise measuring autotaxin by utilizing the aptamer of the present invention (e.g., by the use of the complex and solid phase carrier of the present invention). The method of detecting and quantifying autotaxin can be performed in the same manner as an immunological method, except that the aptamer of the present invention is used in place of an antibody. Therefore, by using the aptamer of the present invention as a probe in place of an antibody, in the same manner as such methods as enzyme immunoassay (EIA) (e.g., direct competitive ELISA, indirect competitive ELISA, sandwich ELISA), radioimmunoassay (RIA), fluorescent immunoassay (FIA), Western blot method, immunohistochemical staining method, and cell sorting method, detection and quantitation can be performed. It can also be used as a molecule probe for PET and the like. These methods can be useful in, for example, measuring autotaxin contents in living organisms or biological samples, and in diagnosing a disease associated with autotaxin.

The disclosures in all publications mentioned herein, including patents and patent application specifications, are incorporated by reference herein in the present invention to the extent that all of them have been given expressly.

The present invention is explained in more detail in the following by referring to Examples, which are not to be construed as limitative.

[Examples]

Example 1: Production of DNA aptamer that specifically binds to autotaxin - 1

Using a random sequence of 40 nucleotides as a DNA template, a partly-improved SELEX method (method of Fitter et al., Stephen Fitter and Robert James, J. Biol. Chem., VOL. 280, NO. 40, pp. 34193-34201, October 7, 2005) was performed. As a target substance of SELEX, His-tagged autotaxin (Recombinant Human, manufactured by R&D) immobilized on TALON Metal Affinity Resin (manufactured by Clontech) as a carrier was used. The sequences of the templates and primers used are shown below. A random pool of DNAs and primers were produced by chemical synthesis.

The DNAs bound to autotaxin were amplified by PCR, and treated with exonuclease (BioLabs) to give single strand DNAs. Thereafter, they were treated with λ exonuclease (BioLabs) to convert double stranded DNAs to single strand DNAs, and the single strand DNAs were used as a pool for the next round.

DNA random pool sequence: 5'-GTGGTCTAGCTGTACTCNNNNNNNNNNNNNNNNNNNNNNNNNNNNNNNNNNNNNNNNCCACAG TCAACGAGCTA-3' (SEQ ID NO: 1)
Primer Fwd: 5'-GTGGTCTAGCTGTACTC-3' (SEQ ID NO: 2)
Primer Rev: 5'-p-TAGCTCGTTGACTGTGG-3' (SEQ ID NO: 3)

N in the DNA random pool sequence (SEQ ID NO: 1) is any combination of deoxyribonucleotides (A, G, C or T). In addition, primer Rev having been phosphorylated (p) at 5'-terminus was used.

After 7 rounds of SELEX, the sequences of 51 clones were examined to find convergence in the sequences. The sequences thereof are shown in SEQ ID NOs: 4 - 10. Of these, 10 sequences had the sequence shown in SEQ ID NO: 4, 19 sequences had the sequence shown in SEQ ID NO: 5, 2 sequences had the sequence shown in SEQ ID NO: 6, 2 sequences had the sequence shown in SEQ ID NO: 7, 4 sequences had the sequence shown in SEQ ID NO: 8, 1 sequence had the sequence shown in SEQ ID NO: 9, and 2 sequences had the sequence shown in 10. These sequences contained the following common sequence. Of these sequences, the secondary structure prediction of a clone having a nucleotide sequence shown in SEQ ID NOs: 4 and 5 is shown in Fig. 1A. A possible common secondary structure 1 of the following common subsequence is shown in Fig. 1B. In Fig. 1A, the nucleotides of the common subsequence contained in these clones are enclosed in a circle (O). The nucleotides corresponding to $X_1$ - $X_4$ are enclosed in a dotted line circle (O).

Each nucleotide sequence is shown below. Unless particularly indicated, the sequences shown below are in the 5' to 3' direction, and all are deoxyribonucleotides.

SEQ ID NO: 4:

GTGGTCTAGCTGTACTCTCCGGAACCAGAGCAATTTGGTCGAGCGCTATCGGATGGTCCACAG
TCAACGAGCTA

SEQ ID NO: 5:

GTGGTCTAGCTGTACTCATGGACGGAACCAGAATACTTTTGGTCTCCATTGAGTACGCCACAG
TCAACGAGCTA

SEQ ID NO: 6:

GTGGTCTAGCTGTACTCGGAACCGTACTCAACGGTCAGTACCTTTGCGCCGCAGCAAGCCACA
GTCAACGAGCTA

SEQ ID NO: 7:

GTGGTCTAGCTGTACTCGCCTGCCGGAACCGCCCCTGTGGTCGCATCGAGCAACGGCCCACAG
TCAACGAGCTA

SEQ ID NO: 8:

GTGGTCTAGCTGTACTCCGAAAGCCGGAACCGTGCCAATGGTCGCTACTTCAGCTCCCCACAG
TCAACGAGCTA

SEQ ID NO: 9:

GTGGTCTAGCTGTACTCAGGCCGGAACCGGTGAAATTGGTCGCCTAATAAGCGAAATCCACAG
TCAACGAGCTA

SEQ ID NO: 10:

GTGGTCTAGCTGTACTCGCCGGAACCGTACTATGGTCGCGTTGTATGACGCTTGTATCCACAG

TCAACGAGCTA

common sequence: -$X_3X_1$CGGAACC-$N_1$-GGTC$X_2X_4$ ($N_1$ shows any of 7 to 11 nucleotides, $X_1$, $X_2$, $X_3$ and $X_4$ are each any nucleotide, and at least one of $X_1$ and $X_2$, and $X_3$ and $X_4$ forms Watson-Crick base pairs)-

All nucleic acids shown in SEQ ID NOs: 4 - 10 were produced by chemical synthesis. The binding activity of these nucleic acids to autotaxin was evaluated by the surface plasmon resonance method. For the measurement, Biacore T100 manufactured by GE Healthcare was used. The SA chips with streptavidin immobilized thereon were used as the sensor chips. Binding thereto was about 1500 RU of 16 nucleotide Poly dT of which biotin was bound to the 5'-terminus. The nucleic acids used as a ligand were added with Poly A (16 nucleotides) to the 3'-terminus, and were immobilized on SA chips by T-A annealing. The nucleic acids (20 μL) were injected at a flow rate of 20 μL/min to immobilize about 1500 RU of the nucleic acids. An autotaxin for analyte was prepared at 0.02 μM, and 20 μL thereof was injected. As a running buffer, solution A (mixed solution of 145 mM sodium chloride, 5.4 mM potassium chloride, 1.8 mM calcium chloride, 0.8 mM magnesium chloride, 20 mM Tris (pH 7.6), 0.05% Tween20) was used.

The measurement results are shown in Table 1. As a result of the measurement, it was found that SEQ ID NOs: 4 - 10 bind to autotaxin. The nucleic acid pool (40N) shown in SEQ ID NO: 1, which was used for the first round and contained a 40-nucleotide random sequence used as a negative control, was not more than 10% of SEQ ID NO: 10 that showed the highest binding amount, and was found to not bind thereto (indicated as "-"). The binding amount here shows the maximum Resonance Unit (RU) value.

Whether these nucleic acids show an autotaxin inhibitory activity was evaluated by the following method. As a substrate of autotaxin, phosphodiester bond-containing synthetic substrate p-nitrophenyl thymidine 5'-monophosphate (pNP-TMP) (SIGMA) was selected (hereinafter to be referred to as NPP2 inhibitory assay). A phosphodiester bond is cleaved by hydrolysis, and p-nitrophenol is liberated. The p-nitrophenol develops a yellow color, and the color is detected. For the assay, a 96-well plate (96-Well EIA/RIA Polystyrene Plates, Costar) was used, and the amount of the reaction mixture was 200 μL. As the reaction mixture, solution A was used. Nucleic acids were prepared in solution A (100 μL), pNP-TMP (20 μL) adjusted to 10 mM in the reaction mixture A was added, and the mixture was stirred well and heated at 37°C for 5 min. On the other hand, 6 ng of autotaxin diluted with solution A was prepared (80 μL), and heated at 37°C for 5 min. After heating, they were mixed to start enzyme reaction. The final autotaxin concentration in the reaction solution was 0.3 nM, and the final substrate concentration was 1 mM. A plate containing the reaction mixture was heated at 37°C for 24 hr, placed in a microplate reader SpectraMax190 (manufactured by Molecular Devices) and the absorbance was determined at wavelength 405 nm. The absorbance when nucleic acids are not added as 100% (A0), an inhibitory rate was determined from the absorbance (A) of each test substance and according to the following formula.

$$\text{Enzyme activity rate} = (A/A0) \times 100$$

The concentration ($IC_{50}$) of an inhibitor necessary for inhibiting the enzyme activity by 50% was determined. The results thereof are shown in Table 1. When 40N nucleic acid pool was used as a control (negative control), a similar treatment was also performed, and the measurement was conducted. As a result, the aptamers shown in SEQ ID NOs: 4 - 10 showed high inhibitory activities as evidenced by the $IC_{50}$ values of not more than 100 nM.

Table 1

| Binding activity to autotaxin and NPP2 inhibitory activity ($IC_{50}$ value) | | |
|---|---|---|
| SEQ ID NO: | Binding activity by Biacore | NPP2 inhibitory assay $IC_{50}$ value (μM) |
| 1 (40N) | - | >1.0 |
| 4 | + | 0.016±0.0060 |
| 5 | + | 0.011±0.0050 |
| 6 | + | 0.020±0.000 |
| 7 | + | 0.01210.0030 |
| 8 | + | 0.010±0.0020 |
| 9 | + | 0.036±0.0040 |
| 10 | + | 0.02110.0060 |

"-" shows not more than 10% of the aptamer shown in SEQ ID NO: 10 that showed the highest binding amount, and "+" shows not less than that. The binding amount here shows the maximum Resonance Unit (RU) value. The $IC_{50}$ value shows mean of 2 - 3 measurements$\pm$standard deviation, and ">1.0" indicates that an inhibitory activity was not found in the concentration range up to 1.0 $\mu$M.

Example 2: Chain shortening and base substitution of aptamers

An aptamer having the nucleotide sequence shown in SEQ ID NO: 5 was subjected to chain shortening and base substitution. The sequences of the altered forms are shown in SEQ ID NOs: 11 - 16. Of these, the secondary structure prediction of the aptamers shown in SEQ ID NOs: 11 and 12 is shown in Fig. 2. In the Figure, the nucleotides of the common subsequence are enclosed in a circle (O). The nucleotides corresponding to $X_1$ - $X_4$ are enclosed in a dotted line circle (O). Unless particularly indicated, the sequences shown below are in the 5' to 3' direction, and all are deoxyribonucleotides.

SEQ ID NO: 11 (sequence after chain shortening of aptamer shown in SEQ ID NO: 5 to length of 45 nucleotides including the common sequence) GTACTCATGGACGGAACCAGAATACTTTTGGTCTCCATTGAGTAC

SEQ ID NO: 12 (sequence after chain shortening of aptamer shown in SEQ ID NO: 5 to length of 34 nucleotides including the common sequence and substitution of nucleotides at 3 sites) CCTGGACGGAACCAGAATACTTTTGGTCTCCAGG

SEQ ID NO: 13 (sequence after chain shortening of aptamer shown in SEQ ID NO: 5 to length of 30 nucleotides including the common sequence) TGGACGGAACCAGAATACTTTTGGTCTCCA

SEQ ID NO: 14 (sequence after chain shortening of aptamer shown in SEQ ID NO: 5 to length of 30 nucleotides including the common sequence and substitution of nucleotides at 2 sites) GGGACGGAACCAGAATACTTTTGGTCTCCC

SEQ ID NO: 15 (sequence after chain shortening of aptamer shown in SEQ ID NO: 5 to length of 30 nucleotides including the common sequence) CCTGGACGGAACCAATACTTGGTCTCCAGG

SEQ ID NO: 16 (sequence after chain shortening of aptamer shown in SEQ ID NO: 5 to length of 32 nucleotides including the common sequence and substitution of nucleotides at 2 sites) CTGGACGGAACCAGAATACTTTTGGTCTCCAG

All nucleic acids of SEQ ID NOs: 11 - 16 were produced by chemical synthesis. Whether these nucleic acids bind to autotaxin was evaluated by the surface plasmon resonance method. For the measurement, Biacore T100 manufactured by GE Healthcare was used, and the measurement was performed by the method shown below. About 2700 RU autotaxin was immobilized on a sensorchip surface of CM4 chip by using an amino coupling kit. The flow rate was 20 $\mu$L/min, and nucleic acids (20 $\mu$L) prepared to 0.3 $\mu$M were injected as an analyte. As a running buffer, solution A was used.

The measurement results are shown in Table 2. In Table 2, nucleic acids showing a binding amount of not more than 10% of that of the aptamer having the nucleotide sequence shown in SEQ ID NO: 12 were considered not binding and marked with (-), and ones not less than that were considered binding and marked with (+). The binding amount here shows the maximum Resonance Unit (RU) value. As a result, it was found that the aptamers having the nucleotide sequences shown in SEQ ID NOs: 11 - 14 and 16 bind to autotaxin.

Whether these nucleic acids show an autotaxin inhibitory activity was measured by a method similar to that in Example 1. The $IC_{50}$ values thereof are shown in Table 2. As a result of NPP2 inhibitory assay, the aptamers shown in SEQ ID NOs: 11 - 14 and 16 showed a high inhibitory activity as evidenced by the $IC_{50}$ value of not more than 100 nM.

From the results of SEQ ID NO: 12 contained in Table 2, it was found that the inhibitory activity was maintained even when the length was 34 nucleotides and 3 sites were substituted. From the results of SEQ ID NO: 14 which was SEQ ID NO: 13 in which T at the 5'-terminus was substituted by G and A at the 3'-terminus was substituted by C, it was also found that chain shortening to 30 nucleotides was possible by partial substitution. It was also found that SEQ ID NO: 13 having the common sequential part has a secondary structure different from the common secondary structure 1, due to which the activity of SEQ ID NO: 13 was considered to have markedly decreased, though the activity was present.

Furthermore, SEQ ID NO: 15 lacked the upper stem of the common secondary structure 1, due to which the activity was considered to have markedly decreased.

Table 2

| Binding activity to autotaxin and NPP2 inhibitory activity ($IC_{50}$ value) | | | |
|---|---|---|---|
| SEQ ID NO: | length | Binding activity by Biacore | NPP2 inhibitory assay $IC_{50}$ value ($\mu$M) |
| 11 | 45 | + | $0.012 \pm 0.000$ |
| 12 | 34 | + | $0.012 \pm 0.000$ |
| 13 | 30 | + | $0.11 \pm 0.0070$ |
| 14 | 30 | + | $0.042 \pm 0.0050$ |
| 15 | 30 | - | $0.99 \pm 0.011$ |
| 16 | 32 | + | $0.018 \pm 0.0010$ |

"-" shows not more than 10% of the aptamer shown in SEQ ID NO: 12 that showed the highest binding amount, and "+" shows not less than that. The binding amount here shows the maximum Resonance Unit (RU) value. The $IC_{50}$ value shows mean of 2 - 3 measurements$\pm$standard deviation.

Example 3: Production of DNA aptamer that specifically binds to autotaxin - 2

Using a random sequence of 40 nucleotides, which was different from that in Example 1, as a DNA template, SELEX was performed in the same manner as in Example 1. The sequences of the templates and primers used are shown below. The templates of DNA and primer were produced by chemical synthesis.
DNA random pool sequence: 5'-ACACTCACAGGCGCTGGNNNNNNNNNNNNNNNNNNNNNNNNNNNNNNCGTGCATGGCCGCTAG T-3': (SEQ ID NO: 17)
primer Fwd: 5'-ACACTCACAGGCGCTGG-3': (SEQ ID NO: 18)
primer Rev: 5'-p-ACTAGCGGCCATGCACG-3': (SEQ ID NO: 19)

N in the DNA random pool (SEQ ID NO: 17) is any combination of deoxyribonucleotides (A, G, C or T). In addition, primer Rev having been phosphorylated (p) at 5'-terminus was used.
After 8 rounds of SELEX, the sequences of 90 clones were examined to find convergence of sequences having the above-mentioned common sequence. The sequences thereof are shown in SEQ ID NOs: 20 - 25. Of these, 2 sequences had the sequence shown in SEQ ID NO: 20, 3 sequences had the sequence shown in SEQ ID NO: 21, 19 sequences had the sequence shown in SEQ ID NO: 22, 2 sequences had the sequence shown in SEQ ID NO: 23, 3 sequences had the sequence shown in SEQ ID NO: 24, and 3 sequences had the sequence shown in 25. Unless particularly indicated, the sequences shown below are in the 5' to 3' direction, and all are deoxyribonucleotides.
SEQ ID NO: 20:

ACACTCACAGGCGCTGGGGTACGCTCGGAACCGAGGCAATTGGTCAGCGTGCATGGCCGCTAG
T

SEQ ID NO: 21:

ACACTCACAGGCGCTGGCCACCACTGCACCGGAACCGCGAATGTGGTCGTGCATGGCCGCTAG
T

SEQ ID NO: 22:

ACACTCACAGGCGCTGGCCGGAACCGTGCATATGGTCGCCAGCACATCGTGCATGGCCGCTAG
T

SEQ ID NO: 23:

ACACTCACAGGCGCTGGCACGGACCGGAACCGGGACGCTCGGTCGACCGTGCATGGCCGCTAG T

SEQ ID NO: 24:

ACACTCACAGGCGCTGGCGAGTCGGAACCGAGCCGATTGGTCACTCGCGTGCATGGCCGCTAG T

SEQ ID NO: 25:

ACACTCACAGGCGCTGGCGACGTCGGAACCGTGTACCATGGTCACGTCGTGCATGGCCGCTAG T

All nucleic acids shown in SEQ ID NOs: 20 - 25 were produced by chemical synthesis. The binding activity of these nucleic acids to autotaxin was evaluated by the surface plasmon resonance method similar to that in Example 2. The measurement results are shown in Table 3. As a result of the measurement, it was found that SEQ ID NOs: 20 - 25 bind to autotaxin. The nucleic acid pool (40N), which was used for the first round and contained a 40-nucleotide random sequence used as a negative control, was not more than 10% of SEQ ID NO: 22 that showed the highest binding amount, and was found to not bind thereto (indicated as "-"). The binding amount here shows the maximum Resonance Unit (RU) value.

Whether these nucleic acids show an autotaxin inhibitory activity was measured by a method similar to that in Example 1. The $IC_{50}$ values thereof are shown in Table 3. As a result of NPP2 inhibitory assay, the aptamers shown in SEQ ID NOs: 20 - 25 showed a high inhibitory activity as evidenced by the $IC_{50}$ value of not more than 100 nM.

Table 3

| Binding activity to autotaxin and NPP2 inhibitory activity ($IC_{50}$ value) | | |
|---|---|---|
| SEQ ID NO: | Binding activity by Biacore | NPP2 inhibitory assay $IC_{50}$ value (μM) |
| 17 (40N) | - | >1.0 |
| 20 | + | 0.037±0.0020 |
| 21 | + | 0.028±0.011 |
| 22 | + | 0.030±0.011 |
| 23 | + | 0.075±0.000 |
| 24 | + | 0.032±0.0042 |
| 25 | + | 0.031±0.0057 |

"-" shows not more than 10% of the aptamer shown in SEQ ID NO: 22 that showed the highest binding amount, and "+" shows not less than that. The binding amount here shows the maximum Resonance Unit (RU) value. The $IC_{50}$ value shows mean of 2 - 3 measurements±standard deviation, and ">1.0" indicates that an inhibitory activity was not found in the concentration range up to 1.0 μM.

Example 4: Chain shortening of aptamer

SEQ ID NOs: 20 and 22 were subjected to chain shortening. The altered forms of the sequences are shown in SEQ ID NOs: 26 - 29.

Unless particularly indicated, the sequences shown below are in the 5' to 3' direction, and all are deoxyribonucleotides.

SEQ ID NO: 26 (sequence after chain shortening of aptamer shown in SEQ ID NO: 20 to length of 38 nucleotides including the common sequence): CGCTGGGGTACGCTCGGAACCGAGGCAATTGGTCAGCG

SEQ ID NO: 27 (sequence after chain shortening of aptamer shown in SEQ ID NO: 20 to length of 32 nucleotides including the common sequence): TACGCTCGGAACCGAGGCAATTGGTCAGCGTG

SEQ ID NO: 28 (sequence after chain shortening of aptamer shown in SEQ ID NO: 22 to length of 31 nucleotides including the common sequence): ACAGGCGCTGGCCGGAACCGTGCATATGGTC

SEQ ID NO: 29 (sequence after chain shortening of aptamer shown in SEQ ID NO: 22 to length of 31 nucleotides including the common sequence): GCTGGCCGGAACCGTGCATATGGTCGCCAGC

All nucleic acids shown in SEQ ID NOs: 26 - 29 were produced by chemical synthesis. Whether these nucleic acids show an autotaxin inhibitory activity was measured by a method similar to that in Example 1. The $IC_{50}$ values thereof are shown in Table 4. As a result, the aptamers shown in SEQ ID NOs: 27 and 29 showed a high inhibitory activity as evidenced by the $IC_{50}$ value of not more than 100 nM.

While SEQ ID NOs: 26 and 28 contained the common sequence, they were different from the common secondary structure 1, and their inhibitory activities decreased markedly (Table 4). On the contrary, SEQ ID NOs: 27 and 29 subjected to chain shortening to have the common secondary structure were found to show high inhibitory activities.

[Table 4]

| NPP2 inhibitory activity against autotaxin ($IC_{50}$ value) | | |
|---|---|---|
| SEQ ID NO: | Length | NPP2 inhibitory assay $IC_{50}$ value (μM) |
| 26 | 38 | 0.44±0.041 |
| 27 | 32 | 0.024±0.010 |
| 28 | 31 | >1.0 |
| 29 | 31 | 0.011±0.0040 |

The $IC_{50}$ value shows mean of 2 - 3 measurements±standard deviation, and ">1.0" indicates that an inhibitory activity was not found in the concentration range up to 1.0 μM.

Example 5: Modification of chain-shortened aptamer

An altered form of the aptamer shown in SEQ ID NO: 12 having a modified terminal, and an altered form wherein modification has been introduced into the 2'-position of ribose of purine nucleotide in the sequence were produced. The sequences thereof are shown in SEQ ID NOs: 12(1) - 12(148). All nucleic acids were produced by chemical synthesis. Unless particularly indicated, respective sequences shown below are deoxyribonucleotides shown in the 5' to 3' direction. The parenthesis in the nucleotide shows modification at the 2'-position, and M shows a methoxy group. U shows uracil, and a lower case letter s shows phosphorothioation. Nj and N(M)j (N is A, G, C or T) shows P-methylated nucleotide, and P-methylated and 2'-methoxylated nucleotide, respectively (see the following structural formula).

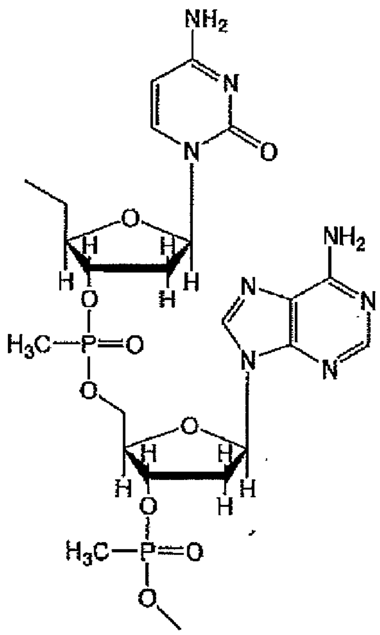

P-methyl nucleotide (CjAj)

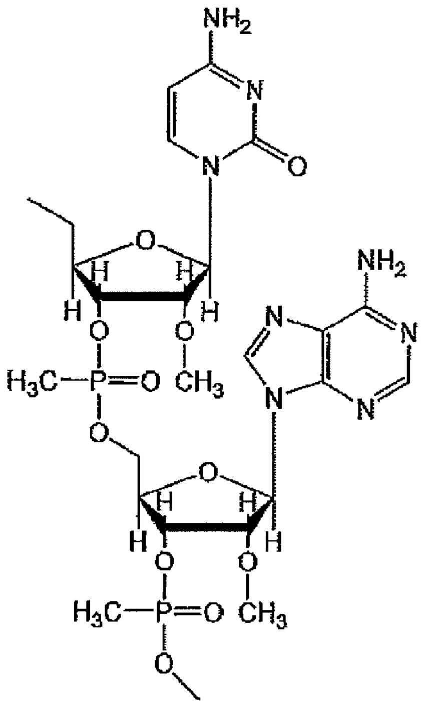

P-methyl-2'methoxy nucleotide (C(M)jA(M)j)

idT in terminal modification shows inverted-dT, and C12 or 6 shows spacer C12 or 6. Furthermore, Y shows ssH linker. In addition, 40P shows SUNBRIGHT GL2-400GS2, 80P shows SUNBRIGHT GL2-800GS2, 40PP shows SUNBRIGHT GL2-400TS, 80PP shows SUNBRIGHT GL2-800TS, 80PPP shows SUNBRIGHT GL4-800GS2, 40PPPP shows SUNBRIGHT GL4-400TS, and 80PPPP shows SUNBRIGHT GL4-800TS, each of which is polyethylene glycol.

SEQ ID NO: 12(1): (sequence of aptamer shown in SEQ ID NO: 12 with methoxy-modification introduced thereinto) C(M)C(M)T(M)GGACGGAACCAGAATACTTTTGGTCTCCAGG

SEQ ID NO: 12(2): (sequence of aptamer shown in SEQ ID NO: 12 with methoxy-modification introduced thereinto) CCTGGACGGAACCAGAATACTTTTGGTCTC(M)C(M)AGG

SEQ ID NO: 12(3): (sequence of aptamer shown in SEQ ID NO: 12 with methoxy-modification introduced thereinto) CCTGGACGGAACCAGAATACTTTTGGTCTCCA(M)G(M)G(M)

SEQ ID NO: 12(4): (sequence of aptamer shown in SEQ ID NO: 12 with methoxy-modification introduced thereinto) CCTGGACGGAAC(M)C(M)AGAATACTTTTGGTCTCCAGG

SEQ ID NO: 12(5): (sequence of aptamer shown in SEQ ID NO: 12 with methoxy-modification introduced thereinto) CCTGGACGGAACCAGAATACTTT(M)T(M)GGTCTCCAGG

SEQ ID NO: 12(6): (sequence of aptamer shown in SEQ ID NO: 12 with methoxy-modification introduced thereinto) CCTGGACGGAACCAGAATACTTTTG(M)G(M)TCTCCAGG

SEQ ID NO: 12(7): (sequence of aptamer shown in SEQ ID NO: 12 with methoxy-modification introduced thereinto) CCTGGACGGAACCA(M)G(M)AATACTTTTGGTCTCCAGG

SEQ ID NO: 12(8): (sequence of aptamer shown in SEQ ID NO: 12 with methoxy-modification introduced thereinto) CCTGGA(M)CGGAACCAGAATACTTTTGGTCTCCAGG

SEQ ID NO: 12(9): (sequence of aptamer shown in SEQ ID NO: 12 with methoxy-modification introduced thereinto) CCTGGACGGAACCAGAATACTTTTGGTCT(M)CCAGG

SEQ ID NO: 12(10): (sequence of aptamer shown in SEQ ID NO: 12 with methoxy-modification introduced thereinto) CCTGGACGGAA(M)CCAGAATACTTTTGGTCTCCAGG

SEQ ID NO: 12(11): (sequence of aptamer shown in SEQ ID NO: 12 with methoxy-modification introduced thereinto) CCTGGACGGAACCAGAATACTTTTGGT(M)CTCCAGG

SEQ ID NO: 12(12): (sequence of aptamer shown in SEQ ID NO: 12 with methoxy-modification introduced thereinto) CCTGGAC(M)GGAACCAGAATACTTTTGGTCTCCAGG

SEQ ID NO: 12(13): (sequence of aptamer shown in SEQ ID NO: 12 with methoxy-modification introduced thereinto) CCTGGACG(M)GAACCAGAATACTTTTGGTCTCCAGG

SEQ ID NO: 12(14): (sequence of aptamer shown in SEQ ID NO: 12 with methoxy-modification introduced thereinto) CCTGGACGG(M)AACCAGAATACTTTTGGTCTCCAGG

SEQ ID NO: 12(15): (sequence of aptamer shown in SEQ ID NO: 12 with methoxy-modification introduced thereinto) CCTGGACGGA(M)ACCAGAATACTTTTGGTCTCCAGG

SEQ ID NO: 12(16): (sequence of aptamer shown in SEQ ID NO: 12 with methoxy-modification introduced thereinto) CCTGGACGGAACCAGAA(M)TACTTTTGGTCTCCAGG

SEQ ID NO: 12(17): (sequence of aptamer shown in SEQ ID NO: 12 with methoxy-modification introduced thereinto) CCTGGACGGAACCAGAAT(M)ACTTTTGGTCTCCAGG

SEQ ID NO: 12(18): (sequence of aptamer shown in SEQ ID NO: 12 with methoxy-modification introduced thereinto) CCTGGACGGAACCAGAATA(M)CTTTTGGTCTCCAGG

SEQ ID NO: 12(19): (sequence of aptamer shown in SEQ ID NO: 12 with methoxy-modification introduced thereinto) CCTGGACGGAACCAGAATAC(M)TTTTGGTCTCCAGG

SEQ ID NO: 12(20): (sequence of aptamer shown in SEQ ID NO: 12 with methoxy-modification introduced thereinto) CCTGGACGGAACCAGAATACT(M)TTTGGTCTCCAGG

SEQ ID NO: 12(21): (sequence of aptamer shown in SEQ ID NO: 12 with methoxy-modification introduced thereinto) CCTG(M)G(M)ACGGAACCAGAATACTTTTGGTCTCCAGG

SEQ ID NO: 12(22): (sequence of aptamer shown in SEQ ID NO: 12 with idT introduced into both terminals) idT-CCTGGACGGAACCAGAATACTTTTGGTCTCCAGG-idT

SEQ ID NO: 12(23): (sequence of aptamer shown in SEQ ID NO: 12(22) with 40kDa polyethylene glycol introduced thereinto instead of 5'-terminal idT) 40P-Y-CCTGGACGGAACCAGAATACTTTTGGTCTCCAGG-idT

SEQ ID NO: 12(24): (sequence of aptamer shown in SEQ ID NO: 12(22) with methoxy-modification introduced thereinto)

idT-CCTGGAC(M)GG(M)AACCA(M)G(M)AATA(M)C(M)TTTTGGTCTCCA(M)G(M)G(M)-idT

SEQ ID NO: 12(25): (sequence of aptamer shown in SEQ ID NO: 12(22) with methoxy-modification introduced thereinto)

idT-C(M)C(M)T(M)G(M)G(M)AC(M)GG(M)AAC(M)C(M)A(M)G(M)AA(M)T(M)A(M)C(M)TTTTGGTCTCCA(M)G(M)G(M)-idT

SEQ ID NO: 12(26): (sequence of aptamer shown in SEQ ID NO: 12 with methoxy-modification introduced thereinto)
CCTGGACGGAACCAGAATACTTTTGGTC(M)TCCAGG
SEQ ID NO: 12(27): (sequence of aptamer shown in SEQ ID NO: 12 with methoxy-modification introduced thereinto)
CCTGGACGGAACCAGA(M)ATACTTTTGGTCTCCAGG
SEQ ID NO: 12(28): (sequence of aptamer shown in SEQ ID NO: 12 with methoxy-modification introduced thereinto)
CCTGGACGGAACCAGAATACTT(M)TTGGTCTCCAGG
SEQ ID NO: 12(29): (sequence of aptamer shown in SEQ ID NO: 12 with methoxy-modification introduced thereinto)
CCTGGACGGAACCAGAATACTTTTGG(M)TCTCCAGG
SEQ ID NO: 12(30): (sequence of aptamer shown in SEQ ID NO: 12 with methoxy-modification introduced thereinto)
CCTGGACGGAACCAGAATACTTTTG(M)GTCTCCAGG
SEQ ID NO: 12(31): (sequence of aptamer shown in SEQ ID NO: 12 with methoxy-modification introduced thereinto)
CCTGGACGGAAC(M)CAGAATACTTTTGGTCTCCAGG
SEQ ID NO: 12(32): (sequence of aptamer shown in SEQ ID NO: 12 with methoxy-modification introduced thereinto)
CCTGGACGGAACC(M)AGAATACTTTTGGTCTCCAGG
SEQ ID NO: 12(33): (sequence of aptamer shown in SEQ ID NO: 12 with methoxy-modification introduced thereinto)
CCTGGACGGAACCAGAATACTTTT(M)GGTCTCCAGG
SEQ ID NO: 12(34): (sequence of aptamer shown in SEQ ID NO: 12 with methoxy-modification introduced thereinto)
CCTGGACGGAACCAGAATACTTT(M)TGGTCTCCAGG
SEQ ID NO: 12(35): (sequence of aptamer shown in SEQ ID NO: 12 with methoxy-modification introduced thereinto)
CCTG(M)GACGGAACCAGAATACTTTTGGTCTCCAGG
SEQ ID NO: 12(36): (sequence of aptamer shown in SEQ ID NO: 12 with methoxy-modification introduced thereinto)
CCTGG(M)ACGGAACCAGAATACTTTTGGTCTCCAGG
SEQ ID NO: 12(37): (sequence of aptamer shown in SEQ ID NO: 12 with methoxy-modification introduced thereinto)
CCTGGACGGAACCAGAATACTTTTGGTCTC(M)CAGG
SEQ ID NO: 12(38): (sequence of aptamer shown in SEQ ID NO: 12 with methoxy-modification introduced thereinto)
CCTGGACGGAACCAGAATACTTTTGGTCTCC(M)AGG
SEQ ID NO: 12(39): (sequence of aptamer shown in SEQ ID NO: 12 with methoxy-modification introduced thereinto)
CCT(M)GGACGGAACCAGAATACTTTTGGTCTCCAGG
SEQ ID NO: 12(40): (sequence of aptamer shown in SEQ ID NO: 12 with methoxy-modification introduced thereinto)
CC(M)TGGACGGAACCAGAATACTTTTGGTCTCCAGG
SEQ ID NO: 12(41): (sequence of aptamer shown in SEQ ID NO: 12 with methoxy-modification introduced thereinto)
C(M)CTGGACGGAACCAGAATACTTTTGGTCTCCAGG
SEQ ID NO: 12(42): (sequence of aptamer shown in SEQ ID NO: 12(22) with methoxy-modification introduced thereinto)

idT-C(M)CTGG(M)AC(M)GG(M)AAC(M)C(M)A(M)G(M)AATA(M)C(M)TTTTGGTCTCCA(M)G(M)G(M)-idT

SEQ ID NO: 12(43): (sequence of aptamer shown in SEQ ID NO: 12(22) with methoxy-modification introduced thereinto)

idT-C(M)CTGG(M)AC(M)GG(M)AAC(M)CA(M)G(M)AATA(M)C(M)TTTTGGTCTCCA(M)G(M)G(M)-idT

SEQ ID NO: 12(44): (sequence of aptamer shown in SEQ ID NO: 12(22) with methoxy-modification introduced thereinto)

idT-

C(M)CTGG(M)AC(M)GG(M)AACC(M)A(M)G(M)AATA(M)C(M)TTTTGGTCTCCA(M)G

(M)G(M)-idT

SEQ ID NO: 12(45): (sequence of aptamer shown in SEQ ID NO: 12(22) with methoxy-modification introduced thereinto)

idT-

C(M)CTGGAC(M)GG(M)AAC(M)CA(M)G(M)AATA(M)C(M)TTTTGGTCTCCA(M)G(M)

G(M)-idT

SEQ ID NO: 12(46): (sequence of aptamer shown in SEQ ID NO: 12(22) with methoxy-modification introduced thereinto)

idT-

C(M)CTGGAC(M)GG(M)AACC(M)A(M)G(M)AATA(M)C(M)TTTTGGTCTCCA(M)G(M)

G(M)-idT

SEQ ID NO: 12(47): (sequence of aptamer shown in SEQ ID NO: 12(22) with methoxy-modification introduced thereinto)

idT-

C(M)CTGG(M)AC(M)GG(M)AAC(M)C(M)A(M)G(M)AATA(M)C(M)TTTTGGTCTC(M)

CA(M)G(M)G(M)-idT

SEQ ID NO: 12(48): (sequence of aptamer shown in SEQ ID NO: 12(24) with 40kDa polyethylene glycol introduced thereinto instead of 5'-terminal idT)

40P-Y-

CCTGGAC(M)GG(M)AACCA(M)G(M)AATA(M)C(M)TTTTGGTCTCCA(M)G(M)G(M)-

idT

SEQ ID NO: 12(49): (sequence of aptamer shown in SEQ ID NO: 12(42) with 40kDa polyethylene glycol introduced thereinto instead of 5'-terminal idT)

40P-Y-

C(M)CTGG(M)AC(M)GG(M)AAC(M)C(M)A(M)G(M)AATA(M)C(M)TTTTGGTCTCCA(

M)G(M)G(M)-idT

SEQ ID NO: 12(50): (sequence of aptamer shown in SEQ ID NO: 12(24) with 80kDa polyethylene glycol introduced thereinto instead of 5'-terminal idT)

80P-Y-
CCTGGAC(M)GG(M)AACCA(M)G(M)AATA(M)C(M)TTTTGGTCTCCA(M)G(M)G(M)-
idT

SEQ ID NO: 12(51): (sequence of aptamer shown in SEQ ID NO: 12(24) with 80kDa polyethylene glycol which is different from SEQ ID NO: 12(50), instead of 5'-terminal idT)

80PP-Y-
CCTGGAC(M)GG(M)AACCA(M)G(M)AATA(M)C(M)TTTTGGTCTCCA(M)G(M)G(M)-
idT

SEQ ID NO: 12(52): (sequence of aptamer shown in SEQ ID NO: 12(24) with 80kDa polyethylene glycol which is different from SEQ ID NO: 12(50) and 12(51), instead of 5'-terminal idT)

80PPP-Y-
CCTGGAC(M)GG(M)AACCA(M)G(M)AATA(M)C(M)TTTTGGTCTCCA(M)G(M)G(M)-
idT

SEQ ID NO: 12(53): (sequence of aptamer shown in SEQ ID NO: 12(24) with 80kDa polyethylene glycol which is different from SEQ ID NO: 12(50)-12(52), instead of 5'-terminus idT)

80PPPP-Y-
CCTGGAC(M)GG(M)AACCA(M)G(M)AATA(M)C(M)TTTTGGTCTCCA(M)G(M)G(M)-
idT

SEQ ID NO: 12(54): (sequence of aptamer shown in SEQ ID NO: 12(24) with C12 introduced thereinto instead of 3'-terminal idT)

idT-
CCTGGAC(M)GG(M)AACCA(M)G(M)AATA(M)C(M)TTTTGGTCTCCA(M)G(M)G(M)-
C12

SEQ ID NO: 12(55): (sequence of aptamer shown in SEQ ID NO: 12(24) with C12 introduced thereinto instead of 5'-terminal idT)

C12-
CCTGGAC(M)GG(M)AACCA(M)G(M)AATA(M)C(M)TTTTGGTCTCCA(M)G(M)G(M)-
idT

SEQ ID NO: 12(56):(sequence of aptamer shown in SEQ ID NO: 12(48) with C6 introduced thereinto instead of 3'-terminal idT)

40P-Y-
CCTGGAC(M)GG(M)AACCA(M)G(M)AATA(M)C(M)TTTTGGTCTCCA(M)G(M)G(M)-
C6

SEQ ID NO: 12(57): (sequence of aptamer shown in SEQ ID NO: 12(48) with C12 introduced thereinto instead of 3'-terminus idT)

40P-Y-
CCTGGAC(M)GG(M)AACCA(M)G(M)AATA(M)C(M)TTTTGGTCTCCA(M)G(M)G(M)-
C12

SEQ ID NO: 12(58): (sequence of aptamer shown in SEQ ID NO: 12(24), wherein one site is substituted by P-methylnucleotide)

idT-
CCjTGGAC(M)GG(M)AACCA(M)G(M)AATA(M)C(M)TTTTGGTCTCCA(M)G(M)G(M)-
idT

SEQ ID NO: 12(59): (sequence of aptamer shown in SEQ ID NO: 12(24), wherein one site is substituted by P-methylnucleotide)

idT-
CCTGjGAC(M)GG(M)AACCA(M)G(M)AATA(M)C(M)TTTTGGTCTCCA(M)G(M)G(M)-
idT

SEQ ID NO: 12(60): (sequence of aptamer shown in SEQ ID NO: 12(24), wherein one site is substituted by P-methylnucleotide)

idT-
CCTGGAC(M)GG(M)AACCA(M)G(M)AATjA(M)C(M)TTTTGGTCTCCA(M)G(M)G(M)-
idT

SEQ ID NO: 12(61): (sequence of aptamer shown in SEQ ID NO: 12(24), wherein one site is substituted by P-methylnucleotide)

idT-
CCTGGAC(M)GG(M)AACjCA(M)G(M)AATA(M)C(M)TTTTGGTCTCCA(M)G(M)G(M)-
idT

SEQ ID NO: 12(62): (sequence of aptamer shown in SEQ ID NO: 12(24), wherein one site is substituted by P-methylnucleotide)

idT-CCTGGAC(M)GG(M)AACCA(M)G(M)AATA(M)C(M)TTTTGGTCjTCCA(M)G(M)G(M)-idT

SEQ ID NO: 12(63): (sequence of aptamer shown in SEQ ID NO: 12(24), wherein one site is substituted by P-methylnucleotide.)

idT-CCTGGAC(M)GG(M)AACCA(M)G(M)AATA(M)C(M)TTTTGGTCTCCAjG(M)G(M)-idT

SEQ ID NO: 12(64): (sequence of aptamer shown in SEQ ID NO: 12(24), wherein one site is substituted by P-methylnucleotide)

idT-CCTGGAC(M)GG(M)AACCA(M)GjAATA(M)C(M)TTTTGGTCTCCA(M)G(M)G(M)-idT

SEQ ID NO: 12(65): (sequence of aptamer shown in SEQ ID NO: 12(24), wherein one site is substituted by P-methylnucleotide)

idT-CCTGGAC(M)GG(M)AACCA(M)G(M)AATA(M)CjTTTTGGTCTCCA(M)G(M)G(M)-idT

SEQ ID NO: 12(66): (sequence of aptamer shown in SEQ ID NO: 12(48) with 40kDa polyethylene glycol which is different from SEQ ID NO: 12(48) introduced into 5'-terminus)

40PP-Y-CCTGGAC(M)GG(M)AACCA(M)G(M)AATA(M)C(M)TTTTGGTCTCCA(M)G(M)G(M)-idT

SEQ ID NO: 12(67): (sequence of aptamer shown in SEQ ID NO: 12(48) with 40kDa polyethylene glycol which is different from SEQ ID NO: 12(48) and SEQ ID NO: 12(66) introduced into 5'-terminus)

40PPPP-Y-CCTGGAC(M)GG(M)AACCA(M)G(M)AATA(M)C(M)TTTTGGTCTCCA(M)G(M)G(M)-idT

SEQ ID NO: 12(68): (sequence of aptamer shown in SEQ ID NO: 12(24) wherein T at one site is substituted by U(M))

idT-CCU(M)GGAC(M)GG(M)AACCA(M)G(M)AATA(M)C(M)TTTTGGTCTCCA(M)G(M)G(M)-idT

SEQ ID NO: 12(69): (sequence of aptamer shown in SEQ ID NO: 12(24) wherein T at one site is substituted by U(M))

idT-CCTGGAC(M)GG(M)AACCA(M)G(M)AATA(M)C(M)TTTU(M)GGTCTCCA(M)G(M)G(M)-idT

SEQ ID NO: 12(70): (sequence of aptamer shown in SEQ ID NO: 12(24) wherein T at one site is substituted by U(M))

idT-CCTGGAC(M)GG(M)AACCA(M)G(M)AATA(M)C(M)TTU(M)TGGTCTCCA(M)G(M)G(M)-idT

SEQ ID NO: 12(71): (sequence of aptamer shown in SEQ ID NO: 12(24) wherein T at one site is substituted by U(M))

idT-CCTGGAC(M)GG(M)AACCA(M)G(M)AATA(M)C(M)TTTTGGTCU(M)CCA(M)G(M)G(M)-idT

SEQ ID NO: 12(72): (sequence of aptamer shown in SEQ ID NO: 12(24) wherein T at one site is substituted by U(M))

idT-CCTGGAC(M)GG(M)AACCA(M)G(M)AATA(M)C(M)TTTTGGU(M)CTCCA(M)G(M)G(M)-idT

SEQ ID NO: 12(73): (sequence of aptamer shown in SEQ ID NO: 12(24) wherein T at one site is substituted by U(M))

idT-CCTGGAC(M)GG(M)AACCA(M)G(M)AATA(M)C(M)TU(M)TTGGTCTCCA(M)G(M)G(M)-idT

SEQ ID NO: 12(74): (sequence of aptamer shown in SEQ ID NO: 12(24) wherein T at one site is substituted by U(M))

idT-CCTGGAC(M)GG(M)AACCA(M)G(M)AATA(M)C(M)U(M)TTTGGTCTCCA(M)G(M)G(M)-idT

SEQ ID NO: 12(75): (sequence of aptamer shown in SEQ ID NO: 12(24) wherein T at one site is substituted by U(M))

idT-CCTGGAC(M)GG(M)AACCA(M)G(M)AAU(M)A(M)C(M)TTTTGGTCTCCA(M)G(M)G(M)-idT

SEQ ID NO: 12(76): (sequence of aptamer shown in SEQ ID NO: 12(24) with phosphorothioate introduced thereinto)

idT-CCTGGAsC(M)GG(M)AACCA(M)G(M)AATA(M)C(M)TTTTGGTCTCCA(M)G(M)G(M)-idT

SEQ ID NO: 12(77): (sequence of aptamer shown in SEQ ID NO: 12(24) with phosphorothioate introduced thereinto)

idT-CCTGGAC(M)GsG(M)AACCA(M)G(M)AATA(M)C(M)TTTTGGTCTCCA(M)G(M)G(M)-idT

SEQ ID NO: 12(78): (sequence of aptamer shown in SEQ ID NO: 12(24) with phosphorothioate introduced thereinto)

idT-CCTGGAC(M)GG(M)AsACCA(M)G(M)AATA(M)C(M)TTTTGGTCTCCA(M)G(M)G(M)-idT

SEQ ID NO: 12(79): (sequence of aptamer shown in SEQ ID NO: 12(24) with phosphorothioate introduced thereinto)

idT-CCTGGAC(M)GG(M)AAsCCA(M)G(M)AATA(M)C(M)TTTTGGTCTCCA(M)G(M)G(M)-idT

SEQ ID NO: 12(80): (sequence of aptamer shown in SEQ ID NO: 12(24) with phosphorothioate introduced thereinto)

idT-CCTGGAC(M)GG(M)AACsCA(M)G(M)AATA(M)C(M)TTTTGGTCTCCA(M)G(M)G(M)-idT

SEQ ID NO: 12(81): (sequence of aptamer shown in SEQ ID NO: 12(24) with phosphorothioate introduced thereinto)

idT-CCTGGAC(M)GG(M)AACCsA(M)G(M)AATA(M)C(M)TTTTGGTCTCCA(M)G(M)G(M)-idT

SEQ ID NO: 12(82): (sequence of aptamer shown in SEQ ID NO: 12(24) with phosphorothioate introduced thereinto)

idT-CCTGGAC(M)GG(M)AACCA(M)G(M)AATA(M)C(M)TTTTsGGTCTCCA(M)G(M)G(M)-idT

SEQ ID NO: 12(83): (sequence of aptamer shown in SEQ ID NO: 12(24) with phosphorothioate introduced thereinto)

idT-CCTGGAC(M)GG(M)AACCA(M)G(M)AATA(M)C(M)TTTTGsGTCTCCA(M)G(M)G(M)-idT

SEQ ID NO: 12(84): (sequence of aptamer shown in SEQ ID NO: 12(24) with phosphorothioate introduced thereinto)

idT-CCTGGAC(M)GG(M)AACCA(M)G(M)AATA(M)C(M)TTTTGGsTCTCCA(M)G(M)G(M)-idT

SEQ ID NO: 12(85): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTsCTCCA(M)G(M)G(M)-idT

SEQ ID NO: 12(86): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTCsTCCA(M)G(M)G(M)-idT

SEQ ID NO: 12(87): (sequence of aptamer shown in SEQ ID NO: 12(24) with phosphorothioate introduced thereinto)

idT-CCTGGAC(M)GG(M)AACCA(M)G(M)AATA(M)C(M)TTTsTGGTCTCCA(M)G(M)G(M)-idT

SEQ ID NO: 12(88): (sequence of aptamer shown in SEQ ID NO: 12(24) wherein two sites are substituted by P-methylnucleotide)

idT-CCTGGAC(M)GG(M)AACjCA(M)G(M)AATA(M)C(M)TTTTGGTCjTCCA(M)G(M)G(M)-idT

SEQ ID NO: 12(89): (sequence of aptamer shown in SEQ ID NO: 12(24), wherein one site is substituted by P-methylnucleotide)

idT-CCTGGAC(M)GG(M)AACCA(M)G(M)AATA(M)C(M)TTTTGGjTCTCCA(M)G(M)G(M)-idT

SEQ ID NO: 12(90): (sequence of aptamer shown in SEQ ID NO: 12(24), wherein one site is substituted by P-methylnucleotide)

idT-

CCTGGAC(M)GG(M)AACCA(M)G(M)AATA(M)C(M)TTTTGjGTCTCCA(M)G(M)G(M)-

idT

SEQ ID NO: 12(91): (sequence of aptamer shown in SEQ ID NO: 12(24), wherein one site is substituted by P-methylnucleotide.)

idT-

CCTGGAC(M)GG(M)AACCjA(M)G(M)AATA(M)C(M)TTTTGGTCTCCA(M)G(M)G(M)-

idT

SEQ ID NO: 12(92): (sequence of aptamer shown in SEQ ID NO: 12(24), wherein one site is substituted by P-methylnucleotide.)

idT-

CCTGGAC(M)GjG(M)AACCA(M)G(M)AATA(M)C(M)TTTTGGTCTCCA(M)G(M)G(M)-

idT

SEQ ID NO: 12(93): (sequence of aptamer shown in SEQ ID NO: 12(24), wherein one site is substituted by P-methylnucleotide.)

idT-

CCTGGAC(M)GG(M)AjACCA(M)G(M)AATA(M)C(M)TTTTGGTCTCCA(M)G(M)G(M)-

idT

SEQ ID NO: 12(94): (sequence of aptamer shown in SEQ ID NO: 12(24), wherein one site is substituted by P-methylnucleotide.)

idT-

CCTGGAC(M)GG(M)AAjCCA(M)G(M)AATA(M)C(M)TTTTGGTCTCCA(M)G(M)G(M)-

idT

SEQ ID NO: 12(95): (sequence of aptamer shown in SEQ ID NO: 12(24), wherein one site is substituted by P-methylnucleotide.)

idT-

CCTGGAjC(M)GG(M)AACCA(M)G(M)AATA(M)C(M)TTTTGGTCTCCA(M)G(M)G(M)-

idT

SEQ ID NO: 12(96): (sequence of aptamer shown in SEQ ID NO: 12(24), wherein one site is substituted by P-methylnucleotide.)

idT-CCTGGAC(M)GG(M)AACCA(M)G(M)AATA(M)C(M)TTTTGGTCTjCCA(M)G(M)G(M)-idT

SEQ ID NO: 12(97): (sequence of aptamer shown in SEQ ID NO: 12(24), wherein one site is substituted by P-methylnucleotide.)

idT-CCTGGAC(M)GG(M)AACCA(M)G(M)AATA(M)C(M)TTTTGGTjCTCCA(M)G(M)G(M)-idT

SEQ ID NO: 12(98): (sequence of aptamer shown in SEQ ID NO: 12(24) with phosphorothioate introduced thereinto)

idT-

CCTGGAC(M)GG(M)AACCA(M)G(M)sAATA(M)C(M)TTTTGGTCTCCA(M)G(M)G(M)-idT

SEQ ID NO: 12(99): (sequence of aptamer shown in SEQ ID NO: 12(24) with phosphorothioate introduced thereinto)

idT-CCTGGAC(M)GG(M)AACCA(M)G(M)AsATA(M)C(M)TTTTGGTCTCCA(M)G(M)G(M)-idT

SEQ ID NO: 12(100): (sequence of aptamer shown in SEQ ID NO: 12(24) with phosphorothioate introduced thereinto)

idT-CCTGGAC(M)GG(M)AACCA(M)G(M)AAsTA(M)C(M)TTTTGGTCTCCA(M)G(M)G(M)-idT

SEQ ID NO: 12(101): (sequence of aptamer shown in SEQ ID NO: 12(24) with phosphorothioate introduced thereinto)

idT-CCTGGAC(M)GG(M)AACCA(M)G(M)AATsA(M)C(M)TTTTGGTCTCCA(M)G(M)G(M)-idT

SEQ ID NO: 12(102): (sequence of aptamer shown in SEQ ID NO: 12(24) with phosphorothioate introduced thereinto)

idT-CCTGGAC(M)GG(M)AACCA(M)G(M)AATA(M)C(M)sTTTTGGTCTCCA(M)G(M)G(M)-idT

SEQ ID NO: 12(103): (sequence of aptamer shown in SEQ ID NO: 12(24) with phosphorothioate introduced thereinto)

idT-CCTGGAC(M)GG(M)AACCA(M)G(M)AATA(M)C(M)TsTTTGGTCTCCA(M)G(M)G(M)-idT

SEQ ID NO: 12(104): (sequence of aptamer shown in SEQ ID NO: 12(24) with phosphorothioate introduced thereinto)

idT-CCTGGAC(M)GG(M)AACCA(M)G(M)AATA(M)C(M)TTsTTGGTCTCCA(M)G(M)G(M)-idT

SEQ ID NO: 12(105): (sequence of aptamer shown in SEQ ID NO: 12(24) with phosphorothioate introduced thereinto)

idT-CCTGGAC(M)GG(M)sAACCA(M)G(M)AATA(M)C(M)TTTTGGTCTCCA(M)G(M)G(M)-idT

SEQ ID NO: 12(106): (sequence of aptamer shown in SEQ ID NO: 12(24) with phosphorothioate introduced thereinto)

idT-CCTGGAC(M)sGG(M)AACCA(M)G(M)AATA(M)C(M)TTTTGGTCTCCA(M)G(M)G(M)-idT

SEQ ID NO: 12(107): (sequence of aptamer shown in SEQ ID NO: 12(24) with phosphorothioate introduced thereinto)

idT-CsCTGGAC(M)GG(M)AACCA(M)G(M)AATA(M)C(M)TTTTGGTCTCCA(M)G(M)G(M)-idT

SEQ ID NO: 12(108): (sequence of aptamer shown in SEQ ID NO: 12(24) with phosphorothioate introduced thereinto)

idT-CCsTGGAC(M)GG(M)AACCA(M)G(M)AATA(M)C(M)TTTTGGTCTCCA(M)G(M)G(M)-idT

SEQ ID NO: 12(109): (sequence of aptamer shown in SEQ ID NO: 12(24) with phosphorothioate introduced thereinto)

idT-CCTsGGAC(M)GG(M)AACCA(M)G(M)AATA(M)C(M)TTTTGGTCTCCA(M)G(M)G(M)-idT

SEQ ID NO: 12(110): (sequence of aptamer shown in SEQ ID NO: 12(24) with phosphorothioate introduced thereinto)

idT-CCTGsGAC(M)GG(M)AACCA(M)G(M)AATA(M)C(M)TTTTGGTCTCCA(M)G(M)G(M)-idT

SEQ ID NO: 12(111): (sequence of aptamer shown in SEQ ID NO: 12(24) with phosphorothioate introduced thereinto)

idT-CCTGGsAC(M)GG(M)AACCA(M)G(M)AATA(M)C(M)TTTTGGTCTCCA(M)G(M)G(M)-idT

SEQ ID NO: 12(112): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTCTsCCA(M)G(M)G(M)-idT

SEQ ID NO: 12(113): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTCTCsCA(M)G(M)G(M)-idT

SEQ ID NO: 12(114): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTCTCCsA(M)G(M)G(M)-idT

SEQ ID NO: 12(115): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTCTCCA(M)sG(M)G(M)-idT

SEQ ID NO: 12(116): (sequence of aptamer shown in SEQ ID NO: 12(24) with phosphorothioate introduced thereinto)

idT-CCTGGAC(M)GG(M)AACCA(M)G(M)AATA(M)C(M)TTTTGGTCTCCA(M)G(M)sG(M)-idT

SEQ ID NO: 12(117): (sequence of aptamer shown in SEQ ID NO: 12(77) with 40kDa polyethylene glycol introduced thereinto instead of 5'-terminal idT)

40P-Y-CCTGGAC(M)GsG(M)AACCA(M)G(M)AATA(M)C(M)TTTTGGTCTCCA(M)G(M)G(M)-idT

SEQ ID NO: 12(118): (sequence of aptamer shown in SEQ ID NO: 12(24), wherein two sites are substituted by P-methylnucleotide)

idT-CCTGGAC(M)GjG(M)AACjCA(M)G(M)AATA(M)C(M)TTTTGGTCTCCA(M)G(M)G(M)-idT

SEQ ID NO: 12(119): (sequence of aptamer shown in SEQ ID NO: 12(24), wherein three sites are substituted by P-methylnucleotide)

idT-CCTGGAC(M)GjG(M)AACjCA(M)G(M)AATA(M)C(M)TTTTGGTCjTCCA(M)G(M)G(M)-idT

SEQ ID NO: 12(120): (sequence of aptamer shown in SEQ ID NO: 12(24), wherein one site is substituted by P-methylnucleotide)

idT-CCTGGAC(M)GG(M)AACCA(M)G(M)AATA(M)C(M)TTTTjGGTCTCCA(M)G(M)G(M)-idT

SEQ ID NO: 12(121): (sequence of aptamer shown in SEQ ID NO: 12(24), wherein one site is substituted by P-methylnucleotide)

idT-CCTGGAC(M)GG(M)AACCA(M)G(M)AATA(M)C(M)TTTjTGGTCTCCA(M)G(M)G(M)-idT

SEQ ID NO: 12(122): (sequence of aptamer shown in SEQ ID NO: 12(24), wherein one site is substituted by P-methylnucleotide)

idT-CCTGGAC(M)GG(M)AACCA(M)G(M)AATA(M)C(M)TTjTTGGTCTCCA(M)G(M)G(M)-idT

SEQ ID NO: 12(123): (sequence of aptamer shown in SEQ ID NO: 12(24), wherein one site is substituted by P-methylnucleotide)

idT-

CCTGGAC(M)GG(M)AACCA(M)G(M)AATA(M)C(M)TjTTTGGTCTCCA(M)G(M)G(M)-

idT

SEQ ID NO: 12(124): (sequence of aptamer shown in SEQ ID NO: 12(24), wherein one site is substituted by P-methylnucleotide)

idT-

CCTGGAC(M)GG(M)AACCA(M)G(M)AjATA(M)C(M)TTTTGGTCTCCA(M)G(M)G(M)-

idT

SEQ ID NO: 12(125): (sequence of aptamer shown in SEQ ID NO: 12(24), wherein one site is substituted by P-methylnucleotide)

idT-

CCTGGAC(M)GG(M)AACCA(M)G(M)AAjTA(M)C(M)TTTTGGTCTCCA(M)G(M)G(M)-

idT

SEQ ID NO: 12(126): (sequence of aptamer shown in SEQ ID NO: 12(24), wherein one site is substituted by P-methylnucleotide)

idT-

CCTGGAC(M)GG(M)AACCA(M)G(M)AATA(M)C(M)TTTTGGTCTCCjA(M)G(M)G(M)-

idT

SEQ ID NO: 12(127): (sequence of aptamer shown in SEQ ID NO: 12(24), wherein one site is substituted by P-methylnucleotide)

idT-

CCTGGAC(M)GG(M)AACCA(M)G(M)AATA(M)C(M)TTTTGGTCTCjCA(M)G(M)G(M)-

idT

SEQ ID NO: 12(128): (sequence of aptamer shown in SEQ ID NO: 12(24), wherein one site is substituted by P-methylnucleotide)

idT-

CCTGGjAC(M)GG(M)AACCA(M)G(M)AATA(M)C(M)TTTTGGTCTCCA(M)G(M)G(M)-

idT

SEQ ID NO: 12(129): (sequence of aptamer shown in SEQ ID NO: 12(24), wherein one site is substituted by P-methylnucleotide)

idT-

CCTjGGAC(M)GG(M)AACCA(M)G(M)AATA(M)C(M)TTTTGGTCTCCA(M)G(M)G(M)-

idT

SEQ ID NO: 12(130): (sequence of aptamer shown in SEQ ID NO: 12(24), wherein one site is substituted by P-methylnucleotide)

idT-

CjCTGGAC(M)GG(M)AACCA(M)G(M)AATA(M)C(M)TTTTGGTCTCCA(M)G(M)G(M)-

idT

SEQ ID NO: 12(131): (sequence of aptamer shown in SEQ ID NO: 12(92) with 40kDa polyethylene glycol introduced thereinto instead of 5'-terminal idT)

40P-Y-

CCTGGAC(M)GjG(M)AACCA(M)G(M)AATA(M)C(M)TTTTGGTCTCCA(M)G(M)G(M)-

idT

SEQ ID NO: 12(132): (sequence of aptamer shown in SEQ ID NO: 12(24), wherein 10 sites are substituted by P-methylnucleotide)

idT-

CjCTGGjAC(M)GjG(M)AACjCA(M)G(M)AjATA(M)C(M)TjTTjTGGTCjTCjCjA(M)

G(M)G(M)-idT

SEQ ID NO: 12(133): (sequence of aptamer shown in SEQ ID NO: 12(119) with 40kDa polyethylene glycol introduced thereinto instead of 5'-terminal idT)

40P-Y-

CCTGGAC(M)GjG(M)AACjCA(M)G(M)AATA(M)C(M)TTTTGGTCjTCCA(M)G(M)G(M

)-idT

SEQ ID NO: 12(134): (sequence of aptamer shown in SEQ ID NO: 12(132) with 40kDa polyethylene glycol introduced thereinto instead of 5'-terminal idT)

40P-Y-

CjCTGGjAC(M)GjG(M)AACjCA(M)G(M)AjATA(M)C(M)TjTTjTGGTCjTCjCjA(M)

G(M)G(M)-idT

SEQ ID NO: 12(135): (sequence of aptamer shown in SEQ ID NO: 12(132), wherein one site is substituted by P-methyl-2'methoxynucleotide)

idT-
CjCTGGjAC(M)GjG(M)AACjCA(M)G(M)AjATA(M)C(M)TjTTjTGGTCjTCjCjA(M)
G(M)G(M)j-idT

SEQ ID NO: 12(136): (sequence of aptamer shown in SEQ ID NO: 12(132), wherein one site is substituted by P-methyl-2'methoxynucleotide)

idT-
CjCTGGjAC(M)GjG(M)AACjCA(M)G(M)AjATA(M)C(M)TjTTjTGGTCjTCjCjA(M)
G(M)jG(M)-idT

SEQ ID NO: 12(137): (sequence of aptamer shown in SEQ ID NO: 12(132), wherein one site is substituted by P-methyl-2'methoxynucleotide)

idT-
CjCTGGjAC(M)GjG(M)AACjCA(M)G(M)AjATA(M)C(M)TjTTjTGGTCjTCjCjA(M)
jG(M)G(M)-idT

SEQ ID NO: 12(138): (sequence of aptamer shown in SEQ ID NO: 12(132), wherein one site is substituted by P-methyl-2'methoxynucleotide)

idT-
CjCTGGjAC(M)GjG(M)AACjCA(M)G(M)AjATA(M)C(M)jTjTTjTGGTCjTCjCjA(M
)G(M)G(M)-idT

SEQ ID NO: 12(139):(sequence of aptamer shown in SEQ ID NO: 12(132), wherein one site is substituted by P-methyl-2'methoxynucleotide)

idT-
CjCTGGjAC(M)GjG(M)AACjCA(M)G(M)AjATA(M)jC(M)TjTTjTGGTCjTCjCjA(M
)G(M)G(M)-idT

SEQ ID NO: 12(140):(sequence of aptamer shown in SEQ ID NO: 12(132), wherein one site is substituted by P-methyl-2'methoxynucleotide.)

idT-
CjCTGGjAC(M)GjG(M)AACjCA(M)G(M)jAjATA(M)C(M)TjTTjTGGTCjTCjCjA(M
)G(M)G(M)-idT

SEQ ID NO: 12(141): (sequence of aptamer shown in SEQ ID NO: 12(132), wherein one site is substituted by P-methyl-2'methoxynucleotide)

idT-
CjCTGGjAC(M)GjG(M)AACjCA(M)jG(M)AjATA(M)C(M)TjTTjTGGTCjTCjCjA(M)G(M)G(M)-idT

SEQ ID NO: 12(142): (sequence of aptamer shown in SEQ ID NO: 12(132), wherein one site is substituted by P-methyl-2'methoxynucleotide)

idT-
CjCTGGjAC(M)GjG(M)jAACjCA(M)G(M)AjATA(M)C(M)TjTTjTGGTCjTCjCjA(M)G(M)G(M)-idT

SEQ ID NO: 12(143): (sequence of aptamer shown in SEQ ID NO: 12(132), wherein one site is substituted by P-methyl-2'methoxynucleotide)

idT-
CjCTGGjAC(M)jGjG(M)AACjCA(M)G(M)AjATA(M)C(M)TjTTjTGGTCjTCjCjA(M)G(M)G(M)-idT

SEQ ID NO: 12(144): (sequence of aptamer shown in SEQ ID NO: 12(135) with 40kDa polyethylene glycol introduced thereinto instead of 5'-terminal idT)

40P-Y-
CjCTGGjAC(M)GjG(M)AACjCA(M)G(M)AjATA(M)C(M)TjTTjTGGTCjTCjCjA(M)G(M)G(M)j-idT

SEQ ID NO: 12(145): (sequence of aptamer shown in SEQ ID NO: 12(139) with 40kDa polyethylene glycol introduced thereinto instead of 5'-terminal idT)

40P-Y-
CjCTGGjAC(M)GjG(M)AACjCA(M)G(M)AjATA(M)jC(M)TjTTjTGGTCjTCjCjA(M)G(M)G(M)-idT

SEQ ID NO: 12(146): (sequence of aptamer shown in SEQ ID NO: 12(141) with 40kDa polyethylene glycol introduced thereinto instead of 5'-terminal idT)

40P-Y-
CjCTGGjAC(M)GjG(M)AACjCA(M)jG(M)AjATA(M)C(M)TjTTjTGGTCjTCjCjA(M)G(M)G(M)-idT

SEQ ID NO: 12(147): (sequence of aptamer shown in SEQ ID NO: 12(134), wherein 3 sites are substituted by P-methyl-2'methoxynucleotide)

40P-Y-CjCTGGjAC(M)GjG(M)AACjCA(M)jG(M)AjATA(M)jC(M)TjTTjTGGTCjTCjCjA(M)G(M)G(M)j-idT

SEQ ID NO: 12(148): (sequence of aptamer shown in SEQ ID NO: 12, subjected to methoxy-modification and introduction of idT into 3'-terminus)

CCTGGAC(M)GG(M)AACCA(M)G(M)AATA(M)C(M)TTTTGGTCTCCA(M)G(M)G(M)-idT

All nucleic acids of SEQ ID NOs: 12(1) - 12(148) were produced by chemical synthesis. Whether these nucleic acids show an autotaxin inhibitory activity was measured.

The amount of the reaction solution was set to 36 μL, and the measurement method was as follows. To human pool serum (manufactured by Kohjin Bio Co., Ltd.) (33 μL) added with 14:0 LPC, prepared with solution A, to a final concentration of 0.5 mM was added an aptamer dissolved in solution A (3 μL) (final serum concentration about 92%), and the mixture was heated at 37°C. After 3 hr, 100 mM EDTA solution (4 μL) was added to discontinue autotaxin activity, and LPA concentration was measured. LPA concentration was measured by the method of Kishimoto et al. (Kishimoto, Clinica Chimica Acta 333, 59-69, 2003). With the concentration of LPA produced in the serum added with solution A instead of aptamer as control (L0), the inhibitory rate of each aptamer was determined from LPA concentration (L) in the serum added with aptamer and according to the following formula.

$$\text{Inhibitory rate} = (L0-L)/L0) \times 100$$

The autotaxin activity inhibitory rates (LPA production inhibitory rate) of samples heated at 37°C for 3 hr are shown in Table 5. The aptamers shown in SEQ ID NOs: 12(1) - 12(4), 12(6) - 12(9), 12(12) - 12(25), 12(27) - 12(41) showed a high inhibitory activity of not less than 50% at a concentration of 5 μM in the LPA assay. The aptamers shown in SEQ ID NOs: 12(42) - 12(49) showed a high inhibitory activity of not less than 50% at a concentration of 1 μM in the LPA assay. The aptamers shown in SEQ ID NOs: 12(50) - 12(57) showed a high inhibitory activity of not less than 50% at a concentration of 0.2 μM in the LPA assay. The aptamers shown in SEQ ID NOs: 12(58) - 12(68), 12(75) - 12(78), 12(80) - 12(88), 12(90) - 12(92), 12(95) - 12(96), 12(98) - 12(104), 12(106) - 12(131) showed a high inhibitory activity of not less than 50% at a concentration of 0.1 μM in the LPA assay. The aptamers shown in SEQ ID NOs: 12(132) - 12(135), 12(137), 12(139), 12(141), 12(144) - 12(147) showed a high inhibitory activity of not less than 50% at a concentration of 0.025 μM in the LPA assay. From the foregoing, it was shown that these aptamers have an inhibitory activity against phospholipase D activity of autotaxin in the serum.

[Table 5-1]

| Inhibitory activity of aptamer against autotaxin | | | | | |
|---|---|---|---|---|---|
| Sequence number | LPA production inhibitory rate (%) | Aptamer addition concentration (μM) | Sequence number | LPA production inhibitory rate (%) | Aptamer addition concentration (μM) |
| 12 (1) | 85 | 5.0 | 12 (75) | 65 | 0.10 |
| 12 (2) | 80 | 5.0 | 12 (76) | 67 | 0.10 |
| 12 (3) | 92 | 5.0 | 12 (77) | 88 | 0.10 |
| 12 (4) | 82 | 5.0 | 12 (78) | 62 | 0.10 |
| 12 (5) | 15 | 5.0 | 12 (79) | 37 | 0.10 |
| 12 (6) | 63 | 5.0 | 12 (80) | 85 | 0.10 |
| 12 (7) | 88 | 5.0 | 12 (81) | 70 | 0.10 |
| 12 (8) | 69 | 5.0 | 12 (82) | 51 | 0.10 |
| 12 (9) | 78 | 5.0 | 12 (83) | 54 | 0.10 |

(continued)

| Inhibitory activity of aptamer against autotaxin | | | | | |
|---|---|---|---|---|---|
| Sequence number | LPA production inhibitory rate (%) | Aptamer addition concentration (μM) | Sequence number | LPA production inhibitory rate (%) | Aptamer addition concentration (μM) |
| 12 (10) | 26 | 5.0 | 12 (84) | 72 | 0.10 |
| 12 (11) | 38 | 5.0 | 12 (85) | 68 | 0.10 |
| 12 (12) | 94 | 5.0 | 12 (86) | 57 | 0.10 |
| 12 (13) | 74 | 5.0 | 12 (87) | 70 | 0.10 |
| 12 (14) | 95 | 5.0 | 12 (88) | 90 | 0.10 |
| 12 (15) | 68 | 5.0 | 12 (89) | 48 | 0.10 |
| 12 (16) | 83 | 5.0 | 12 (90) | 63 | 0.10 |
| 12 (17) | 83 | 5.0 | 12 (91) | 58 | 0.10 |
| 12 (18) | 93 | 5.0 | 12 (92) | 91 | 0.10 |
| 12 (19) | 90 | 5.0 | 12 (93) | 37 | 0.10 |
| 12 (20) | 76 | 5.0 | 12 (94) | 0 | 0.10 |
| 12 (21) | 84 | 5.0 | 12 (95) | 64 | 0.10 |
| 12 (22) | 91 | 5.0 | 12 (96) | 76 | 0.10 |
| 12 (23) | 90 | 5.0 | 12 (97) | 7 | 0.10 |
| 12 (24) | 96 | 5.0 | 12 (98) | 76 | 0.10 |
| 12 (25) | 84 | 5.0 | 12 (99) | 81 | 0.10 |
| 12 (26) | 35 | 5.0 | 12 (100) | 80 | 0.10 |
| 12 (27) | 86 | 5.0 | 12 (101) | 81 | 0.10 |
| 12 (28) | 64 | 5.0 | 12 (102) | 72 | 0.10 |
| 12 (29) | 84 | 5.0 | 12 (103) | 88 | 0.10 |
| 12 (30) | 75 | 5.0 | 12 (104) | 74 | 0.10 |
| 12 (31) | 87 | 5.0 | 12 (105) | 25 | 0.10 |
| 12 (32) | 85 | 5.0 | 12 (106) | 68 | 0.10 |
| 12 (33) | 62 | 5.0 | 12 (107) | 75 | 0.10 |
| 12 (34) | 55 | 5.0 | 12 (108) | 68 | 0.10 |
| 12 (35) | 82 | 5.0 | 12 (109) | 68 | 0.10 |

[Table 5-2]

| Inhibitory activity of aptamer against autotaxin (continued) | | | | | |
|---|---|---|---|---|---|
| Sequence number | LPA production inhibitory rate (%) | Aptamer addition concentration (μM) | Sequence number | LPA production inhibitory rate (%) | Aptamer addition concentration (μM) |
| 12 (36) | 84 | 5.0 | 12 (110) | 63 | 0.10 |
| 12 (37) | 85 | 5.0 | 12 (111) | 60 | 0.10 |
| 12(38) | 83 | 5.0 | 12 (112) | 69 | 0.10 |
| 12 (39) | 85 | 5.0 | 12 (113) | 66 | 0.10 |
| 12 (40) | 84 | 5.0 | 12 (114) | 75 | 0.10 |

(continued)

| Inhibitory activity of aptamer against autotaxin (continued) | | | | | |
|---|---|---|---|---|---|
| Sequence number | LPA production inhibitory rate (%) | Aptamer addition concentration (μM) | Sequence number | LPA production inhibitory rate (%) | Aptamer addition concentration (μM) |
| 12 (41) | 85 | 5.0 | 12 (115) | 77 | 0.10 |
| 12 (42) | 68 | 1.0 | 12 (116) | 72 | 0.10 |
| 12 (43) | 85 | 1.0 | 12 (117) | 90 | 0.10 |
| 12 (44) | 87 | 1.0 | 12 (118) | 98 | 0.10 |
| 12 (45) | 86 | 1.0 | 12 (119) | 97 | 0.10 |
| 12 (46) | 83 | 1.0 | 12 (120) | 67 | 0.10 |
| 12 (47) | 57 | 1.0 | 12 (121) | 80 | 0.10 |
| 12 (48) | 92 | 1.0 | 12 (122) | 65 | 0.10 |
| 12 (49) | 58 | 1.0 | 12 (123) | 74 | 0.10 |
| 12 (50) | 95 | 0.20 | 12 (124) | 76 | 0.10 |
| 12 (51) | 91 | 0.20 | 12 (125) | 78 | 0.10 |
| 12 (52) | 92 | 0.20 | 12 (126) | 77 | 0.10 |
| 12 (53) | 92 | 0.20 | 12 (127) | 79 | 0.10 |
| 12 (54) | 89 | 0.20 | 12 (128) | 78 | 0.10 |
| 12 (55) | 91 | 0.20 | 12 (129) | 73 | 0.10 |
| 12 (56) | 93 | 0.20 | 12 (130) | 77 | 0.10 |
| 12 (57) | 93 | 0.20 | 12 (131) | 91 | 0.10 |
| 12 (58) | 69 | 0.10 | 12 (132) | 59 | 0.025 |
| 12 (59) | 71 | 0.10 | 12 (133) | 90 | 0.025 |
| 12 (60) | 50 | 0.10 | 12 (134) | 69 | 0.025 |
| 12 (61) | 91 | 0.10 | 12 (135) | 50 | 0.025 |
| 12 (62) | 75 | 0.10 | 12 (136) | 17 | 0.025 |
| 12 (63) | 59 | 0.10 | 12 (137) | 56 | 0.025 |
| 12 (64) | 63 | 0.10 | 12 (138) | 44 | 0.025 |
| 12 (65) | 57 | 0.10 | 12 (139) | 71 | 0.025 |
| 12 (66) | 77 | 0.10 | 12 (140) | 29 | 0.025 |
| 12 (67) | 74 | 0.10 | 12 (141) | 56 | 0.025 |
| 12 (68) | 53 | 0.10 | 12 (142) | 44 | 0.025 |

[Table 5-3]

| Inhibitory activity of aptamer against autotaxin (continued) | | | | | |
|---|---|---|---|---|---|
| Sequence number | LPA production inhibitory rate (%) | Aptamer addition concentration (μM) | Sequence number | LPA production inhibitory rate (%) | Aptamer addition concentration (μM) |
| 12 (69) | 2 | 0.10 | 12 (143) | 2 | 0.025 |
| 12 (70) | 9 | 0.10 | 12 (144) | 87 | 0.025 |
| 12 (71) | 31 | 0.10 | 12 (145) | 82 | 0.025 |

(continued)

| Inhibitory activity of aptamer against autotaxin (continued) | | | | | |
|---|---|---|---|---|---|
| Sequence number | LPA production inhibitory rate (%) | Aptamer addition concentration (μM) | Sequence number | LPA production inhibitory rate (%) | Aptamer addition concentration (μM) |
| 12 (72) | 1 | 0.10 | 12 (146) | 70 | 0.025 |
| 12 (73) | 11 | 0.10 | 12 (147) | 70 | 0.025 |
| 12 (74) | 31 | 0.10 | | | |

The LPA production inhibitory rate (%) shows an LPA production inhibitory rate 3 hr after addition of aptamer.

Whether SEQ ID NO: 12(148) shows an autotaxin inhibitory activity was measured by NNP2 inhibitory assay in the same manner as in Example 1. As a result, it was found that it has a high inhibitory activity shown by $IC_{50}$ value of 6.8 nM.

Example 6: Confirmation of specificity of autotaxin aptamer

Whether the aptamer shown in SEQ ID NO: 12(48) has a binding activity to FGF2 (PeproTech) was confirmed by the surface plasmon resonance method. For the measurement, Biacore T100 manufactured by GE Healthcare was used, and the measurement was performed by the method shown below. About 2700 RU autotaxin was immobilized on a sensorchip surface of CM4 chip and FGF2 was immobilized on a flow cell 3 (about 1100 RU) by using an amino coupling kit. With the flow rate of 20 μL/min, nucleic acids (20 μL) prepared to 0.3 μM were injected as an analyte. As a running buffer, solution A was used.

As a result of the measurement, it was found that the aptamer shown in SEQ ID NO: 12(48) does not bind to FGF2 (Fig. 3). This shows that the aptamer of the present invention specifically binds to autotaxin.

Example 7: Effect of autotaxin aptamer on pulmonary fibrosis

The aptamer shown in SEQ ID NO: 12(48), which was produced in Example 4, was intraperitoneally administered to bleomycin-induced pulmonary fibrosis model mice, and the effect thereof was verified.

ICR line SPF mice (10-week-old, male, Charles River Laboratories Japan, Inc.) were intratracheally administered with bleomycin (50 μL) prepared with PBS at 770 μg/mL under anesthesia. From the next day of bleomycin administration, autotaxin aptamer solution dissolved in PBS containing 1 mM magnesium chloride, or PBS containing 1 mM magnesium chloride alone (vehicle group) was intraperitoneally administered once per day at a single dose of 100 μL. The dose of aptamer was two doses of 1 and 3 mg/kg/day. A non-treated control group was also reared for the same test period. At 21 days from the bleomycin administration, the test was completed, the lungs were isolated, and the left lung was cryopreserved for hydroxyproline measurement. Hydroxyproline was measured using Hydroxyproline Colorimetric Assay kit of BioVision, Inc.

The results are shown in Fig. 4. The results are shown by mean of 6 - 7 mice±standard error. Suppression of pathology was found in all aptamer administration groups relative to the vehicle group.

From the above, it was suggested that the aptamer shown in SEQ ID NO: 12(48) is usable as a therapeutic drug for pulmonary fibrosis.

Example 8: Measurement of autotaxin inhibitory activity

Whether the aptamers shown in SEQ ID NOs: 12(144) and 12(149) inhibit lysophospholipase D activity of autotaxin was evaluated by the following method. As a substrate of autotaxin, 14:0 lysophosphatidylcholine (LPC, Avanti) was selected (hereinafter to be referred to as LysoPLD inhibitory assay). LPC is hydrolyzed by the lysophospholipase D activity of autotaxin and decomposed into lysophosphatidic acid (LPA) and choline. Choline is oxidized by choline oxidase to afford hydrogen peroxide. In the presence of the hydrogen peroxide and peroxidase, N-ethyl-N-(2-hydroxy-3-sulfopropyl)-3-methoxyaniline (TOOS) and 4-aminoantipyrine (4-AA) undergo oxidation condensation reaction, which develops a purple color to be detected.

For the reaction, a 96-well plate (polypropylene 96-well, manufactured by BMbio) was used, and the amount of the reaction mixture was 60 μL. As a reaction solution, solution A was used. Nucleic acids were prepared in solution A (20 μL), and 4 mM 14:0 LPC adjusted in solution A (30 μL) was added thereto, and they were thoroughly mixed. 10 μL of autotaxin (12.5 ng) diluted with solution A was prepared and added thereto. A plate containing the reaction mixture was heated at 37°C to start the reaction. The final concentration of autotaxin in the reaction solution was 2.1 nM, and

the final substrate concentration was 2 mM. The lysophospholipase D activity of autotaxin was evaluated as follows. 15 μL of the reaction mixture was placed in a 96-well plate for assay (96-Well EIA/RIA Polystyrene Plates, manufactured by Costar), solution B (150 μL) was added thereto and the mixture was heated at 37°C for 5 min. Solution B is a mixed solution of 100 mM tris (pH 8.0), 0.5 mM TOOS (manufactured by DOJINDO), 10U/mL peroxidase (manufactured by TOYOBO), and 0.01% Triton-X (manufactured by Wako). The absorbance was measured at a wavelength of 548 nm, and used as a blank value. Then, solution C (50 μL) was added, and changes in the absorbance at wavelength 548 nm were measured over time. Solution C contained 100 mM tris (pH 8.0), 10U/mL choline oxidase (manufactured by TOYOBO), 1 mM 4-AA (manufactured by DOJINDO), and 0.01% Triton-X. The absorbance of solution blank at the time point when solution B was added, which had been measured earlier, was subtracted from the absorbance at 15 min after addition of solution C, whereby true absorbance value was obtained. This operation was performed immediately after the start of the reaction (0 hr) and at 6 hr after heating to 37°C, and true absorbance at 0 hr was subtracted from the 6 hr after value to give value (D). With D value without an inhibitor (D0) as 100%, the enzyme activity rate was determined from the following equation, wherein $D_A$ is the value of D when an inhibitor was added.

$$\text{Enzyme activity rate} = (D_A/D0) \times 100$$

The concentration ($IC_{50}$) of an inhibitor necessary for inhibiting the enzyme activity by 50% was determined. The results thereof are shown in Table 6. Table 6 shows LysoPLD inhibitory activity against autotaxin, and shows the LysoPLD inhibitory assay $IC_{50}$ values (nM) of the aptamers shown in SEQ ID NO: 12(144), SEQ ID NO: 12(149), 40N and S32826. The LysoPLD inhibitory assay $IC_{50}$ value (nM) shows mean of 2 - 3 measurements±standard deviation, and ">1000" in the $IC_{50}$ value means that an inhibitory activity was not found in the concentration range up to 1000 nM. From the results shown in Table 6, it was shown that the autotaxin aptamers strongly inhibits lysophospholipase D activity of autotaxin. On the other hand, a similar experiment was performed using 40N negative control nucleic acid pool and low molecular autotaxin inhibitor S32836 (SIGMA). However, an inhibitory activity was not found. SEQ ID NO: 12(149) is shown below.

SEQ ID NO: 12(149): (sequence of aptamer shown in SEQ ID NO: 12(135) wherein P-methyl was removed from one site)

idT-
CCTGGjAC(M)GjG(M)AACjCA(M)G(M)AjATA(M)C(M)TjTTjTGGTCjTCjCjA(M)G
(M)G(M)j-idT

[Table 6]

| SEQ ID NO: | LysoPLD inhibitory assay $IC_{50}$ value (nM) |
|---|---|
| 12(144) | 1.6±0.2 |
| 12 (149) | 6.1±2.8 |
| 40N | >1000 |
| S32826 | >1000 |

Example 9: Modification of aptamer

In Example 5, an altered form wherein only one site is P-methyl-modified was produced, and an inhibitory activity was confirmed in an LPA production inhibitory experiment. As a result, it was found that the activity is improved by applying P-methyl-modification to a phosphoric acid group between the 12th C and the 13th C of the aptamer shown in SEQ ID NO: 12(24). Since the 12th C and the 13th C are in a part of the common sequence, it was assumed that the methyl group directly reacted with autotaxin to improve the inhibitory activity. Therefore, whether introduction of a functional group larger than the methyl group and having high hydrophobicity further improves the inhibitory activity was studied.

An aptamers shown in SEQ ID NO: 12(24) wherein a phosphoric acid group between the 12th C and the 13th

C is modified were produced by chemical synthesis. The sequences thereof are shown in SEQ ID NOs: 12(150) - 12(152). Unless particularly indicated, respective sequences are deoxyribonucleotides shown in the 5' to 3' direction. The parenthesis in the nucleotide shows modification at the 2'-position, and M shows a methoxy group. CαC, CβC and CγC show modification of the phosphoric acid moiety, and show P-isopropoxylation, P-propoxylation, and P-butoxylation, respectively (see the following structural formulas). idT in terminal modification shows inverted-dT.

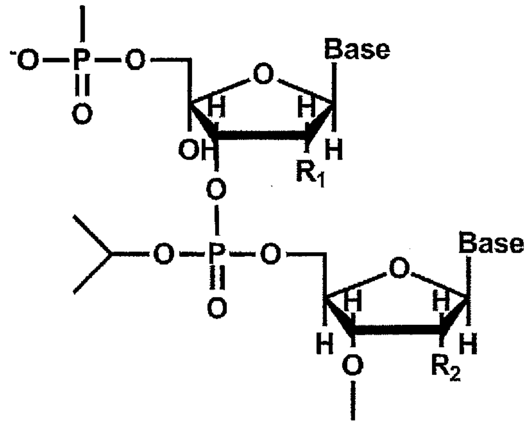

P-isopropoxy nucleotide

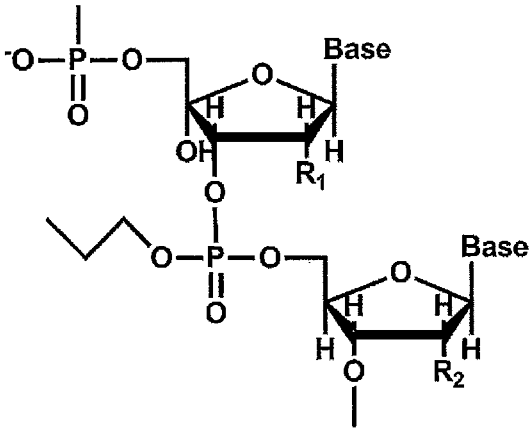

P-propoxy nucleotide

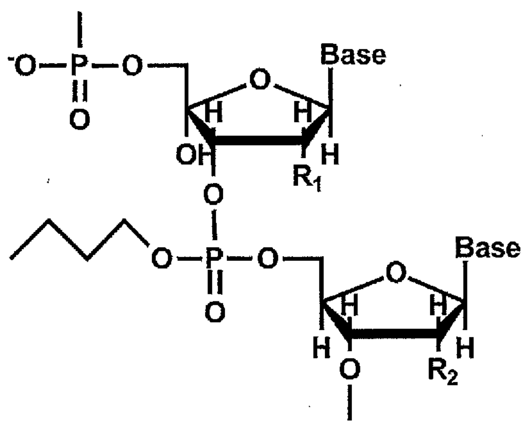

P-butoxy nucleotide

SEQ ID NO: 12(150): sequence of aptamer shown in SEQ ID NO: 12(24) wherein phosphoric acid group between 12th C and 13th C is substituted by P-isopropoxy)

idT-CCTGGAC(M)GG(M)AACαCA(M)G(M)AATA(M)C(M)TTTTGGTCTCCA(M)G(M)G(M)-idT

SEQ ID NO: 12(151): sequence of aptamer shown in SEQ ID NO: 12(24) wherein phosphoric acid group between 12th C and 13th C is substituted by P-propoxy)

idT-CCTGGAC(M)GG(M)AACβCA(M)G(M)AATA(M)C(M)TTTTGGTCTCCA(M)G(M)G(M)-idT

SEQ ID NO: 12(152): sequence of aptamer shown in SEQ ID NO: 12(24) wherein phosphoric acid group between 12th C and 13th C is substituted by P-butoxy)

idT-CCTGGAC(M)GG(M)AACγCA(M)G(M)AATA(M)C(M)TTTTGGTCTCCA(M)G(M)G(M)-idT

Whether the aptamers of SEQ ID NOs: 12(150) - 12(152) show an autotaxin inhibitory activity was examined by NPP2 assay similar to that in Example 1. The $IC_{50}$ values thereof are shown in Table 7. As a result, these aptamers showed a high inhibitory activity shown by $IC_{50}$ value of not more than 1 nM.

[Table 7]

| NPP2 inhibitory activity against autotaxin ($IC_{50}$ value) | |
|---|---|
| SEQ ID NO: | LysoPLD inhibitory assay $IC_{50}$ value (nM) |
| 12(150) | 0.17±0.0013 |
| 12(151) | 0.32±0.0021 |
| 12(152) | 0.23±0.013 |

$IC_{50}$ value shows mean of 2 - 3 measurements±standard deviation.

[Industrial Applicability]

The aptamer or complex of the present invention can be useful as a medicament, or a diagnostic agent or a reagent for diseases such as fibrosis. The aptamer and complex of the present invention can also be useful for the purification and concentration of autotaxin, labeling of autotaxin, as well as detection and quantification of autotaxin.

This application is based on a patent application No. 2014-067289 filed in Japan (filing date: March 27, 2014), the contents of which are incorporated in full herein.

**Claims**

1. An aptamer that binds to an autotaxin, which comprises a nucleotide sequence represented by the following formula (I):

$$CGGAACC\text{-}N_1\text{-}GGTC \qquad (I)$$

wherein $N_1$ shows any of 3 to 11 nucleotides.

2. An aptamer that binds to an autotaxin, which comprises a nucleotide sequence represented by the following formula (II):

$$X_3X_1CGGAACC\text{-}N_1\text{-}GGTCX_2X_4 \qquad (II)$$

wherein $N_1$ shows any of 7 to 11 nucleotides, $X_1$, $X_2$, $X_3$ and $X_4$ are each any nucleotide, and at least one of $X_1$ and $X_2$, and $X_3$ and $X_4$ forms Watson-Crick base pairs.

3. The aptamer according to claim 2, wherein $X_1$ is A, $X_2$ is T, $X_3$ is G, and $X_4$ is C.

**4.** An aptamer that binds to an autotaxin, which comprises a nucleotide sequence shown by the following (a), (b) or (c):

(a) a nucleotide sequence selected from SEQ ID NOs: 4 - 14, 16, 20 - 25, 27 and 29;
(b) a nucleotide sequence selected from SEQ ID NOs: 4 - 14, 16, 20 - 25, 27 and 29, wherein one or several nucleotides other than sequences shown by CGGAACC and GGTC in CGGAACC-$N_1$-GGTC wherein $N_1$ shows any of 3 to 11 nucleotides are substituted, deleted, inserted or added; or
(c) a nucleotide sequence having not less than 70% identity with a nucleotide sequence selected from SEQ ID NOs: 4 - 14, 16, 20 - 25, 27 and 29 (excluding sequences shown by CGGAACC and GGTC in CGGAACC-$N_1$-GGTC wherein $N_1$ is as defined above).

**5.** The aptamer according to any one of claims 1 to 4, wherein $N_1$ is a nucleotide shown by AGAATACTTTT.

**6.** An aptamer that binds to an autotaxin, which has a potential secondary structure represented by the following formula (IV):

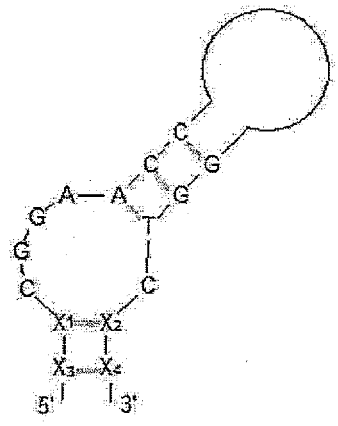


(IV)

wherein $X_1$, $X_2$, $X_3$ and $X_4$ are each any nucleotide, and at least one of $X_1$ and $X_2$, and $X_3$ and $X_4$ forms Watson-Crick base pairs.

**7.** The aptamer according to claim 6, wherein $X_1$ is A, $X_2$ is T, $X_3$ is G, and $X_4$ is C.

**8.** The aptamer according to any one of claims 1 to 7, which has a base length of not less than 30.

**9.** The aptamer according to any one of claims 1 to 8, wherein the hydrogen atoms at the 2'-position of a deoxyribose of respective nucleotides are the same or different and unsubstituted or substituted by an atom or group selected from the group consisting of a fluorine atom and a methoxy group.

**10.** The aptamer according to any one of claims 1 to 9, wherein the phosphoric acid groups contained in the aptamer are the same or different and each is unsubstituted or P-alkylated or P-alkoxylated.

**11.** An aptamer that binds to an autotaxin, which comprises a nucleotide sequence represented by the following formula (I):

CGGAACC-$N_1$-GGTC (I)

wherein $N_1$ shows any of 3 to 11 nucleotides, and
a hydrophobic group is introduced into a phosphoric acid group between C and C of the nucleotide sequence.

**12.** The aptamer according to claim 11, wherein the above-mentioned hydrophobic group is an alkyl group or an alkoxy group.

**13.** The aptamer according to any one of claims 1 to 12, wherein at least one nucleotide is modified.

**14.** The aptamer according to claim 13, which is modified by inverted dT or polyethylene glycol.

**15.** The aptamer according to claim 14, wherein the inverted dT or polyethylene glycol binds to the 5'-terminus or 3'-terminus of the aptamer.

**16.** The aptamer according to any one of claims 1 to 15, wherein at least one phosphoric acid group contained in the aptamer is phosphorothioated or phosphorodithioated.

**17.** A complex comprising the aptamer according to any one of claims 1 to 16 and a functional substance.

**18.** The complex according to claim 17, wherein the functional substance is an affinity substance, a labeling substance, an enzyme, a drug, a toxin or a drug delivery vehicle.

**19.** A medicament comprising the aptamer according to any one of claims 1 to 16, or the complex according to claim 17 or 18.

**20.** An anti-fibrotic agent comprising the aptamer according to any one of claims 1 to 16, or the complex according to claim 17 or 18.

**21.** An autotaxin detection probe, comprising the aptamer according to any one of claims 1 to 16, or the complex according to claim 17 or 18.

**22.** A detection method of autotaxin, comprising using the aptamer according to any one of claims 1 to 16, or the complex according to claim 17 or 18.

Fig. 1

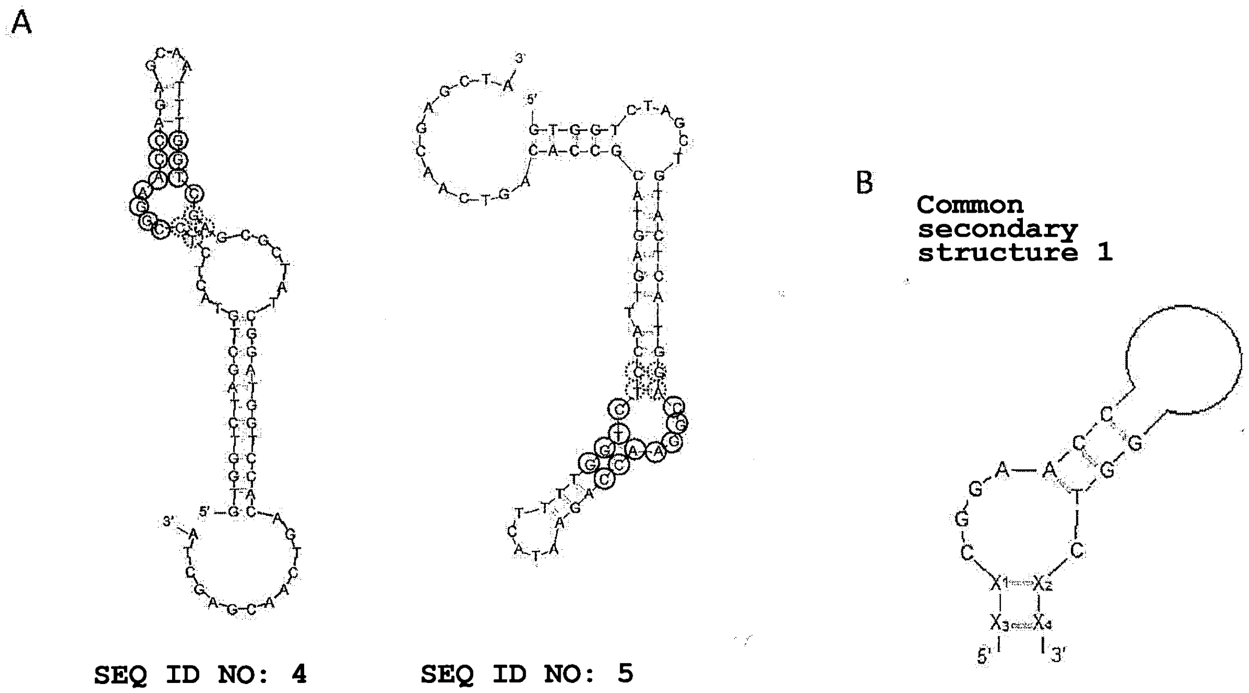


SEQ ID NO: 4

SEQ ID NO: 5

Fig. 2

SEQ ID NO: 11

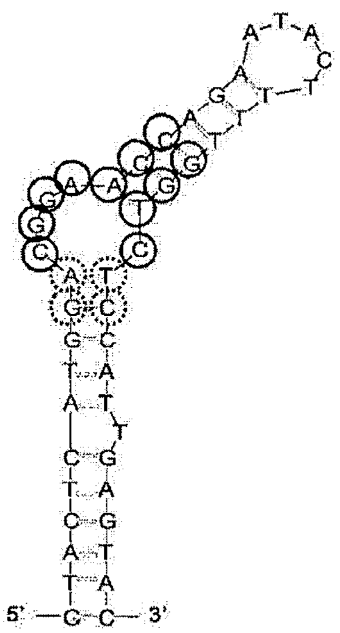


SEQ ID NO: 12

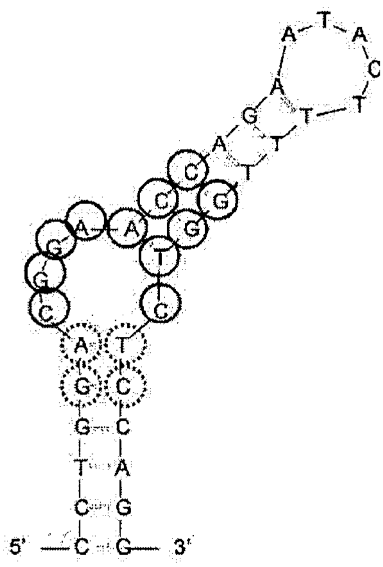

Fig. 3

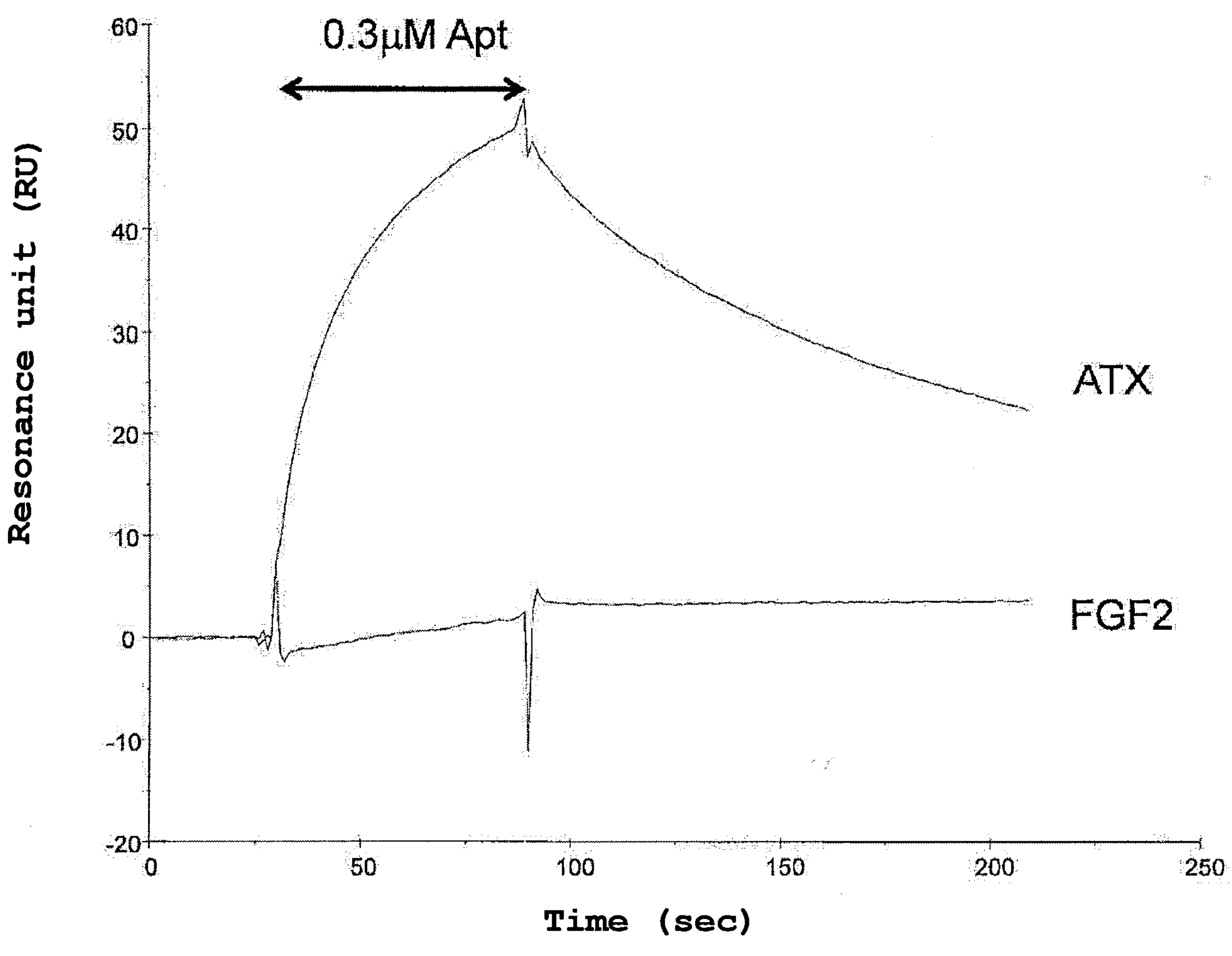

0.3μM Apt
ATX
FGF2
Resonance unit (RU)
Time (sec)
60
50
40
30
20
10
0
-10
-20
0
50
100
150
200
250

Fig. 4

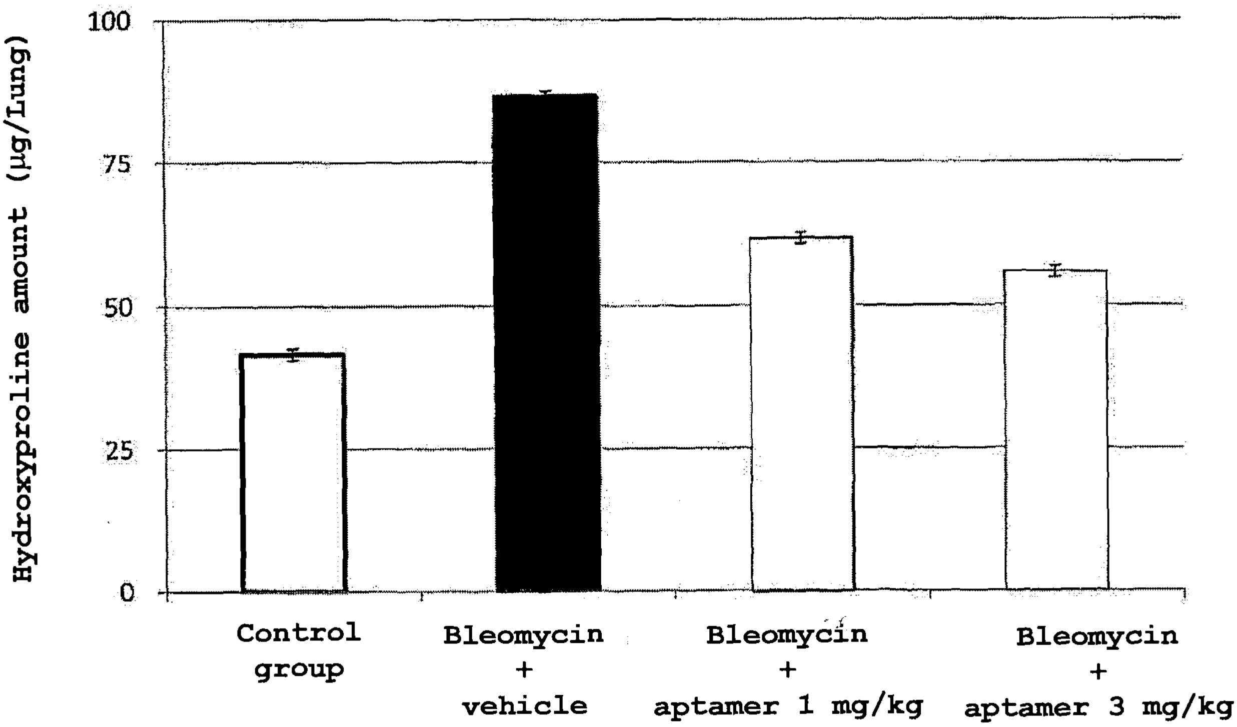
Hydroxyproline amount (μg/Lung)
100
75
50
25
0
Control group
Bleomycin + vehicle
Bleomycin + aptamer 1 mg/kg
Bleomycin + aptamer 3 mg/kg

## INTERNATIONAL SEARCH REPORT

International application No.
PCT/JP2015/059732

A. CLASSIFICATION OF SUBJECT MATTER
*C12N15/00*(2006.01)i, *A61K31/7088*(2006.01)i, *A61K45/00*(2006.01)i, *A61K48/00*(2006.01)i, *A61P43/00*(2006.01)i, *C12N15/115*(2010.01)i, *C12Q1/68*(2006.01)i, *G01N33/53*(2006.01)i, *G01N33/566*(2006.01)i

According to International Patent Classification (IPC) or to both national classification and IPC

B. FIELDS SEARCHED

Minimum documentation searched (classification system followed by classification symbols)
C12N15/00, A61K31/7088, A61K45/00, A61K48/00, A61P43/00, C12N15/115, C12Q1/68, G01N33/53, G01N33/566

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/REGISTRY/WPIDS/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2010/016590 A1 (Nagasaki University, National University Corp.), 11 February 2010 (11.02.2010), & US 2011/0182917 A1 & EP 2324852 A1 | 1-22<br>13-22 |
| X<br>Y | WO 2013/138241 A1 (JANSSEN BIOTECH, INC.), 19 September 2013 (19.09.2013), & US 2015/0087812 A1 & EP 2825199 A | 1-22<br>13-22 |
| X<br>Y | WO 2013/070879 A1 (BRISTOL-MYERS SQUIBB CO.), 16 May 2013 (16.05.2013), (Family: none) | 1-22<br>13-22 |
| X<br>Y | WO 2011/151461 A2 (B.S.R.C. "ALEXANDER FLEMING"), 08 December 2011 (08.12.2011), & US 2013/0202614 A1 & EP 2575794 A | 1-22<br>13-22 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 June 2015 (15.06.15) | 23 June 2015 (23.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/059732

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2013/023040 A2 (LPATH, INC.), 14 February 2013 (14.02.2013), & JP 2014-521723 A & US 2013/0202586 A1 & EP 2741756 A | 1-22<br>13-22 |
| Y | WO 2011/118682 A1 (Ribomic Inc.), 29 September 2011 (29.09.2011), & US 2013/0052176 A1 & EP 2551346 A1 | 13-22 |
| A | WO 2008/016186 A1 (The University of Tokyo), 07 February 2008 (07.02.2008), & US 2010/0120064 A1 & EP 2048501 A1 | 1-22 |
| A | JP 2013-014558 A (Tohoku University), 24 January 2013 (24.01.2013), (Family: none) | 1-22 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5660985 A
- US 5756703 A
- JP 2014067289 A


### Non-patent literature cited in the description


- **FITZWATER ; POLISKY.** *Methods Enzymol.,* 1996, vol. 267, 275-301
- **STEPHEN FITTER ; ROBERT JAMES.** *J. Biol. Chem.,* 2005, vol. 280 (40), 34193-34201
- **SPROAT et al.** *Nucl. Acid. Res.,* 1991, vol. 19, 733-738
- **COTTON et al.** *Nucl. Acid. Res.,* 1991, vol. 19, 2629-2635
- **HOBBS et al.** *Biochemistry,* 1973, vol. 12, 5138-5145
- *Tetrahedron Lett.,* 1997, vol. 38, 8735-8738
- *Tetrahedron,* 2003, vol. 59, 5123-5128
- **RAHMAN S.M.A. ; SEKI S. ; OBIKA S. ; YOSHIKAWA H. ; MIYASHITA K. ; IMANISHI T.** *J. Am. Chem. Soc.,* 2008, vol. 130, 4886-4896
- Synthesis and Analysis of Nucleic Acid. Nucleic Acid. Hirokawa Publishing Company, vol. 2
- **STEPHEN FITTER ; ROBERT JAMES.** *J. Biol. Chem.,* 07 October 2005, vol. 280 (40), 34193-34201
- **KISHIMOTO.** *Clinica Chimica Acta,* 2003, vol. 333, 59-69